# EUROPÄISCHE PATENTANMELDUNG

(11) **EP 2 253 305 A2**
(43) Veröffentlichungstag der Anmeldung: **24.11.2010**
(21) Anmeldenummer: 10161724.9
(22) Anmeldetag: 03.05.2010
(51) Int. Cl.: A61K 8/64, A61K 8/73, A61K 8/97, A61Q 17/04, A61Q 19/08, A61Q 19/00

(54) **Hautbehandlung zur Porenverfeinerung**

(30) Priorität: 22.05.2009 DE 102009026414
(71) Anmelder: Henkel AG & Co. KGaA, 40589 Düsseldorf (DE)
(72) Erfinder: Janßen, Frank, 41470, Neuss (DE); Diickhof, Susanne, 41748, Viersen (DE); Waldmann-Laue, Marianne, 40789, Monheim (DE); Jassoy, Claudia, 40237, Düsseldorf (DE)

(57) **Zusammenfassung**

Die Erfindung betrifft die Verwendung von Reisextrakten sowie Zubereitungen, enthaltend diese in Kombination mit speziellen Inhaltsstoffen zur Verringerung der Porengröße der Haut besitzen.

SEQUENCE LISTING

<110> Henkel AG & Co. KGaA

<120> Hautbehandlung zur Porenverfeinerung

<130> H 08451 EP

<150> DE 102009026414.0
<151> 2009-05-22

<160> 22

<170> PatentIn version 3.3

<210> 1
<211> 4
<212> PRT
<213> Artificial

<220>
<221> MOD_RES
<222> (1)..(1)
<223> N-Palmitoyl-Gly


<400> 2

Gly Gln Pro Arg
1

## Beschreibung

Die Erfindung betrifft die Verwendung von Reisextrakten sowie Zubereitungen, enthaltend diese in Kombination mit speziellen Inhaltsstoffen zur Verringerung der Porengröße der Haut. Große Poren der Haut, insbesondere der Gesichtshaut und des Hals- und Dekollteebereichs sowie des oberen Rückens, werden als unschön und als den Gesamteindruck einer gepflegten Haut störend empfunden. Im Stand der Technik ist bekannt, Aluminium Verbindungen, die adstringierend wirken, einzusetzen. Diese ziehen die Haut an der Oberfläche zusammen und erzeugen so kurzfristig den Eindruck einer kleinporigen Haut. Gleichzeitig entziehen diese Mittel der Haut allerdings Feuchtigkeit, die dringend zur Aufrechterhaltung einer straffen und faltenarmen Haut benötigt wird. Weiterhin werden häufig Zusammensetzungen mit einem deutlichen Anteil von Ethanol eingesetzt. Die Verwendung von ethanolischen Zusammensetzungen führt aber insbesondere bei der nachpubertären Haut häufig zu einer zu starken Entfettung, unangenehmem, spannenden Hautgefühl und zu Reizungen. Es besteht daher nach wie vor das Bedürfnis, große Poren zu bekämpfen und Wirkstoffe oder Wirkstoffkombinationen dafür bereitzustellen, die frei von den genannten Nachteilen sind. In den vergangenen Jahren hat sich bei vielen Kosmetikanwendern, insbesondere bei Frauen in der Zeit nach der Pubertät bis zum Beginn der Wechseljahre, das Phänomen bemerkbar, dass die Probleme mit großporiger Haut über die Pubertät hinaus bestehen, gleichzeitig aber das Erscheinungsbild der Hautalterung immer stärker hervortritt. Pflegeprodukte, die das Erscheinungsbild der Hautalterung, wie insbesondere Fältchen, feine Linien und (erste) Falten, vor allem aber auch den zunehmenden Verlust an Hautfeuchtigkeit, behandeln und mildern sollen, enthalten häufig einen höheren Anteil an pflegenden Fetten und Ölen, die den Feuchtigkeitsgehalt der Haut steigern und die Elastizität der Haut verbessern sollen. Diese Öle und Fette allerdings verschlechtern das Hautbild der Hautpartien, die von Pickeln und Mitessern betroffen sind, welche meist in der sogenannten T-Zone (Stirn, Nase, Kinn) angesiedelt sind. Die Stirn ist allerdings auch besonders von der Bildung von Fältchen und Linien betroffen, so dass deren Behandlung mit einem feuchtigkeitssteigernden Produkt eigentlich zwingend erforderlich wäre.

Die WO 2007/077318 beschreibt die Verwendung eines peptidischen Reisextraktes als aktives Agens, welches die Synthese von SIRT Proteinen in Hautzellen, induziert. EP 0542398 offenbart die Verwendung eines Reisextrakts als Sauerstofffänger. Die US 5853705 beschreibt die Verwendung von hypoallergenem Reisextrakt und UV-Filtern. Die Verwendung von Reisextrakt zur Verringerung der Porengröße der Haut wird in diesen Dokumenten jedoch nicht beschrieben.

Die Aufgabe der vorliegenden Erfindung bestand darin, eine Wirkstoff bereitzustellen, der eine Verringerung der Porengröße der Haut erreicht, ohne der Haut Feuchtigkeit zu entziehen oder sie zu irritieren oder in sonstiger Weise zu belasten. Dabei sollten die Nachteile des Einsatzes bekannter Wirkstoffe, wie Aluminiumverbindungen und Ethanol, vermieden werden können. Insbesondere sollten Hautunverträglichkeiten und -reizungen sowie die Schädigung der hautpositiven Keime in der Hautflora vermieden werden.

Es wurde nun gefunden, dass die Applikation von Reisextrakt zu einer deutlichen Verringerung der Porengröße führt und gleichzeitig äußerst gut verträglich sind.

Gegenstand der vorliegenden Erfindung ist in einer ersten Ausführungsform die kosmetische, nicht-therapeutische Verwendung eines Reisextrakts zur Verringerung der Porengröße der Haut. Insbesondere sind die durch Porenvergrößerung besonders betroffenen Bereiche der Haut, das Gesicht, der Hals- und Dekolleteebereich sowie der obere Rücken umfasst. Insbesondere wird der Reisextrakt auf der Gesichtshaut angewendet.

Als Verringerung der Porengröße wird eine Verkleinerung des Porendurchmessers nach der Behandlung im Vergleich zum Zustand vor der Behandlung verstanden. Gemäß einer bevorzugten Ausführungsform der Erfindung wird die Porengröße um mindestens 3 %, bevorzugt mindestens 5 %, insbesondere um mindestens 7 % und ganz besonders bevorzugt um mindestens 10 % der Ausgangsporengröße verringert wird. Als Verringerung der Porengröße der Haut, insbesondere der Gesichtshaut und des Dekolletees sowie des oberen Rückens, im Sinne der vorliegenden Erfindung ist die Reduktion der Porengröße, insbesondere der durchschnittlichen Porengröße, um mindestens 3 %, bevorzugt mindestens 5 %, insbesondere um mindestens 7 % und ganz besonders bevorzugt um mindestens 10 % der Ausgangsporengröße. Hierbei wird bevorzugt die durchschnittliche Ausgangsporengröße zugrunde gelegt. Die Porengröße wird bevorzugter maßen mit einem konfokalem Laser Scanning Mikroskop bestimmt. Die Messung der Porengröße erfolgt dabei bevorzugt im Gesicht, beispielsweise auf der Nase, dem Kinn, den Wangen und/oder der Stirn. Insbesondere bevorzugt ist die Messung der Porengröße auf der Stirn der Probanden. Die durchschnittliche Ausgangsporengröße wird über das arithmetische Mittel der einzelnen ermittelten Größe der Poren von einem oder bevorzugt mehreren Probanden vor der Behandlung mit einer Zusammensetzung enthaltend den erfindungsgemäß zu verwendenden Extrakt bestimmt.

Gemäß einer weiteren bevorzugten Ausführungsform wird der erfindungsgemäß zu verwendende Reisextrakt durch Extraktion von Reiskörnern, insbesondere ausgewählt aus Körner von Oryza sativa L. und Oryza glaberrima Steud., bevorzugt Oryza sativa L., mit Extraktionsmitteln ausgewählt aus Wasser, wässrige Lösungen, ein- oder mehrfachen Alkoholen mit einer Kettenlänge von 1 bis 6 Kohlenstoffatomen sowie Gemischen aus zwei oder mehreren der genannten Extraktionsmitteln, hergestellt wird. Bevorzugt wird das vollständige Reiskorn eingesetzt, es kann aber auch das geschälte Reiskorn extrahiert werden. Bevorzugte Extraktionsmittel sind Wasser und/oder Alkohole. Unter den Alkoholen sind dabei (C₁ bis C₆)-Alkohole, wie Ethanol und Isopropanol, insbesondere aber mehrwertige Alkohole wie Ethylenglykol, Propylenglykol und Butylenglykol, Glycerin und zwar sowohl als alleiniges Extraktionsmittel als auch in Mischung mit Wasser, bevorzugt. Pflanzenextrakte auf Basis von Wasser/Glycerin im Verhältnis 1:10 bis 10:1 haben sich als besonders geeignet erwiesen. Das Extraktionsmittel enthält bevorzugt mindestens eine Amylase. Diese dient dazu, die überwiegend enthaltene Reisstärke abzubauen, die sonst den Extraktionsprozeß erschwert. Eine besonders bevorzugte Ausführungsform besteht darin, dass der zu verwendende Extrakt Reisproteine und/oder Reisproteinhydrolysate enthält. Besonders bevorzugt enthält der Extrakt vorwiegend Reisproteine, insbesondere Reisproteinhydrolysate. Bevorzugtermaßen enthält der zu verwendende Reisextrakt überwiegend hydrolysierte Reisproteine. Zum Erhalt der Proteinhydrolysate wird bevorzugt eine enzymatische Hydrolyse der Reisproteine in basischem Milieu durchgeführt. Bevorzugt werden dazu Endoproteasen und/oder Exopeptidasen pflanzlichen Ursprungs (Papain, Bromelain, Ficin) oder aus Mikroorganismen (Rhizopus, Bacillus, Aspergillus usw.) eingesetzt. Der erfindungsgemäß verwendete Reisextrakt kann daher bevorzugtermaßen auch als Reisproteinhydrolysat bezeichnet werden. Dabei können Proteinhydrolysate naturgemäß auch monomere Aminosäuren und Oligopeptide enthalten; ihre Zusammensetzung ist normalerweise nicht definiert. Somit ist die Verwendung von Reisproteinhydrolysaten zur Verringerung der Porengröße ebenfalls erfindungsgemäß.

Gemäß einer weiteren besonders bevorzugten Ausführungsform enthält der Reisextrakt mindestens 20 Gew.-%, bevorzugt mindestens 25 Gew.-%, insbesondere mindestens 35 Gew.-% und ganz besonders bevorzugt 41 Gew.-% Peptide (bezogen auf die Trockenmasse des Extrakts) enthält. Die Wirksamkeit eines solchen Extrakts zur Porenverfeinerung ist überraschenderweise besonders gut.

Gemäß einer bevorzugten Ausführungsform der vorliegenden Erfindung wird der Reisextrakt, insbesondere der Reisproteinhydrolysat, bevorzugt der beschriebene peptidische Reisextrakt, in einer Zusammensetzung verwendet, die den Reisextrakt in einer Gesamtmenge von 0,00001 - 10 Gew.-%, bevorzugt 0,0001 - 1 Gew.-%, besonders bevorzugt 0,002 - 0,1 Gew.-%, außerordentlich bevorzugt 0,003 - 0,05 Gew.-%, jeweils bezogen auf den Gehalt an Aktivsubstanz des Extrakts in der gesamten Zusammensetzung enthält.

Der besonders bevorzugt einzusetzende Extrakt Orsirtine® GL von Vincience enthält 0,4 Gew.-% Aktivsubstanz. Der Extrakt wird entweder in seiner Reinform oder bevorzugt zu 0,01 bis 10 Gew.-%, insbesondere zu 0,1 bis 3 Gew.-% Extrakt bezogen auf das Gesamtgewicht der entsprechenden Zusammensetzungen eingesetzt.

Die erfindungsgemäßen bzw. die erfindungsgemäß verwendeten Zusammensetzungen können in verschiedenen Angebotsformen konfektioniert werden, beispielsweise als (ggf. öl- und fettfreies) Gel, als Creme, in Stiftform, als flüssige oder gelförmige Roll on-Applikation, als getränktes flexibles Substrat (Anti-Pickel-Pad), aber auch als Puder oder Spray.

Erfindungsgemäße Mittel können in fester, halbfester, flüssiger, disperser, emulgierter, suspendierter, oder gelförmiger Form vorliegen. Weiterhin können die erfindungsgemäßen Mittel als Aerosol konfektioniert sein, das heißt, sie sind in einem Druckbehälter verpackt, aus dem sie mit Hilfe eines Treibmittels versprüht werden können. Weiterhin können die erfindungsgemäßen Mittel als treibgasfreies Pumpspray versprüht werden.

Ganz besonders bevorzugt werden erfindungsgemäße Mittel als verdickte wäßrige Zusammensetzungen bereitgestellt. Ein die Wirksamkeit der erfindungsgemäßen Kombination besonders unterstützender kosmetischer Träger wird weiter unten offenbart.

Gemäß einer bevorzugten Ausführungsform wird der Reisextrakt in Kombination mit mindestens einem Anti-Aging Wirkstoff verwendet. Insbesondere wird der Reisextrakt, insbesondere das Reisproteinhydrolysat, bevorzugt der bereits genauer beschriebene peptidische Reisextrakt, zur Verringerung der Porengröße gemeinsam eingesetzt mit mindestens ein Anti-Aging-Wirkstoffe, der ausgewählt ist aus Apfelkernextrakten (Pyrus Malus (Apple) Fruit Extract), Hyaluronsäure und/oder ihren Salzen, Lotuskeim-Extrakten (Nelumbo Nucifera Germ Extract), Extrakten aus Maiskörnern (Zea Mays (Corn) Kernel Extract), Extrakten aus Schwarzen Holunderblüten (Sambucus Nigra Flower Extract), Mischungen aus mindestens einem Extrakt aus Kakaobohnen (Theobroma cacao) und mindestens einem Extrakt aus den Blättern der Pfefferminze (Mentha piperita), Resveratrol und/oder Resveratrolmono-, -di- und -triphosphorsäureestern und deren Salzen und/oder Resveratrolmono-, -di- und -trimethylether, Isoflavonoiden und Isoflavonoid-reichen Pflanzenextrakten, Dihydroquercetin (= Taxifolin) sowie Mischungen hiervon, insbesondere aus Hyaluronsäure, einem oder mehreren Salzen der Hyaluronsäure und/oder Apfelkernextrakten. Diese Anti-Aging Wirkstoffe sind in der Lage, die porenverfeinernde noch zu verbessern und gleichzeitig der Haut ein frisches, jugendliches Aussehen zu verleihen.

In einer weiteren bevorzugten Ausführungsform enthalten die erfindungsgemäßen bzw. die erfindungsgemäß verwendeten Zusammensetzungen mindestens einen Extrakt aus Apfelkernextrakten (Pyrus Malus (Apple) Fruit Extract). Erfindungsgemäß besonders bevorzugte Apfelkernextrakte sind unter der Handelsbezeichnung Ederline von der Firma Seporga erhältlich. Das Produkt Ederline enthält Phytohormone, Isoflavonoide, Phytosterole, Triterpenoide, Tocopherole und natürliche Wachse. Ederline ist einmal in wasserlöslicher Form als Ederline-H (INCI: PEG-40 Hydrogenated Castor Oil, PPG-2-Ceteareth-9, Pyrus Malus (Apple) Fruit Extract), zum anderen in fettlöslicher Form als Ederline-L bzw. Ederline-LS (INCI: Hexyldecanol, Pyrus Malus (Apple) Fruit Extract) erhältlich. Erfindungsgemäß besonders bevorzugte kosmetische oder dermatologische Zusammensetzungen sind **dadurch gekennzeichnet, dass** sie den Rohstoff Ederline in Mengen von 0,1 - 10 Gew.-%, bevorzugt 1 - 8 Gew.-% und besonders bevorzugt 3 - 5 Gew.-%, jeweils bezogen auf die gesamte Zusammensetzung, enthalten. Erfindungsgemäß besonders bevorzugt einzusetzende kosmetische oder dermatologische Zusammensetzungen sind **dadurch gekennzeichnet, dass** sie mindestens einen Apfelkernextrakt in Mengen von 0,00001 - 2 Gew.-%, bevorzugt 0,001 - 1,6 Gew.-% und besonders bevorzugt 0,03 - 1 Gew.-%, jeweils bezogen auf den Gehalt an Aktivsubstanz in der gesamten Zusammensetzung, enthalten.

Überraschenderweise zeigte die Kombination aus Apfelkernextrakt und dem Reisextrakt, insbesondere dem Reisproteinhydrolysat, bevorzugt dem bereits vorn definierten peptidischen Reisextrakt, einen synergistischen Effekt auf die Verringerung der Porengröße der Haut. Besonders bevorzugt sind die Kombinationen von 0,001 bis 1,6 Gew.-% Äpfelkernextrakt und 0,0001 bis 2 Gew.-% peptidischem Reisextrakt.

In einer weiteren bevorzugten Ausführungsform enthalten die erfindungsgemäßen bzw. die erfindungsgemäß verwendeten Zusammensetzungen mindestens einen Extrakt aus Lotuskeimen (Nelumbo Nucifera Germ Extract). Derartige Extrakte erhöhen und/oder verbessern die Interaktion zwischen der extrazellulären Matrix und den Fibroblasten. Ein erfindungsgemäß besonders bevorzugter Lotuskeim-Extrakt ist unter der Handelsbezeichnung Lotus Germ Extract mit der INCI-Bezeichnung Water, Butylene Glycol, Nelumbo Nucifera Germ Extract von der Firma Maruzen erhältlich. Erfindungsgemäß besonders bevorzugte kosmetische oder dermatologische Zusammensetzungen sind dadurch gekennzeichnet, dass sie mindestens einen Extrakt aus Lotuskeimen (Nelumbo Nucifera Germ Extract) in Mengen von 0,1 - 10 Gew.-%, bevorzugt 1 - 8 Gew.-% und besonders bevorzugt 2 - 3 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Extrakt aus Lotuskeimen in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen bzw. die erfindungsgemäß verwendeten Zusammensetzungen mindestens einen Extrakt aus Maiskörnern (Zea Mays (Corn) Kernel Extract). Ein erfindungsgemäß besonders bevorzugter Extrakt aus Maiskörnern ist unter der Handelsbezeichnung Deliner von der Firma Coletica erhältlich. Dieser Extrakt erhöht und/oder verbessert die Interaktion zwischen der extrazellulären Matrix und den Fibroblasten. Erfindungsgemäß besonders bevorzugte kosmetische oder dermatologische Zusammensetzungen sind dadurch gekennzeichnet, dass sie mindestens einen Wirkstoff, der ausgewählt ist aus Extrakten aus Maiskörnern (Zea Mays (Corn) Kernel Extract), in einer Gesamtmenge von 0,01 - 5 Gew.-%, bevorzugt 0,1 - 3 Gew.-%, besonders bevorzugt 1 - 2 Gew.-% enthalten, jeweils bezogen auf den Gehalt an Extrakt tel quel in der gesamten Zusammensetzung. Erfindungsgemäß besonders bevorzugte kosmetische oder dermatologische Zusammensetzungen sind **dadurch** gekennzeichnet, dass sie mindestens einen Wirkstoff, der ausgewählt ist aus Extrakten aus Maiskörnern (Zea Mays (Corn) Kernel Extract), in einer Gesamtmenge von 0,00001 - 1 Gew.-%, bevorzugt 0,0001 - 0,1 Gew.-%, besonders bevorzugt 0,001 - 0,05 Gew.-% enthalten, jeweils bezogen auf den Gehalt an Aktivsubstanz in der gesamten Zusammensetzung. In einer weiteren bevorzugten Ausführungsform enthalten die erfindungsgemäßen bzw. die erfindungsgemäß verwendeten Zusammensetzungen mindestens einen Extrakt aus Rotwein. Derartige Extrakte erhöhen und/ oder verbessern die Interaktion zwischen der extrazellulären Matrix und den Fibroblasten. Ein erfindungsgemäß besonders bevorzugter Rotweinextrakt ist unter dem Handelsnamen Sepivinol R von der Firma Seppic erhältlich.

Erfindungsgemäß besonders bevorzugte kosmetische oder dermatologische Zusammensetzungen sind dadurch gekennzeichnet, dass sie mindestens einen Extrakt aus Rotwein in Mengen von 0,1 - 10 Gew.-%, bevorzugt 1 - 8 Gew.-% und besonders bevorzugt 2 - 3 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Extrakt aus Rotwein in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen bzw. die erfindungsgemäß verwendeten Zusammensetzungen mindestens einen Wirkstoff, der ausgewählt ist aus den Mono- und Polyhydroxystilbenen und deren Estern. Unter Polyhydroxystilbenen werden erfindungsgemäß Stilbene verstanden, die mit 2, 3, 4, 5, 6, 7, 8, 9 oder 10 Hydroxygruppen an den beiden Phenylresten substituiert sind, wobei diese verestert sein können. Mono- und Polyhydroxystilbene und deren Ester erhöhen und/oder verbessern die Interaktion zwischen der extrazellulären Matrix und den Fibroblasten. Erfindungsgemäß besonders bevorzugte Hydroxystilbene und deren Ester sind ausgewählt aus Resveratrol (trans-Stilben-3,4'-5-triol), den Resveratrolmono-, -di- und - triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto. Erfindungsgemäß besonders bevorzugte kosmetische oder dermatologische Zusammensetzungen sind dadurch gekennzeichnet, dass sie mindestens einen Wirkstoff, der ausgewählt ist aus den Mono- und Polyhydroxystilbenen und deren Estern, in einer Gesamtmenge von 0,000001 - 5 Gew.-%, bevorzugt 0,00001 - 1 Gew.-%, besonders bevorzugt 0,0001 - 0,1 Gew.-% und außerordentlich bevorzugt 0,005 - 0,05 Gew.-%, enthalten, jeweils bezogen auf den Gehalt an Aktivsubstanz in der gesamten Zusammensetzung. Erfindungsgemäß besonders bevorzugte kosmetische oder dermatologische Zusammensetzungen sind dadurch gekennzeichnet, dass sie mindestens einen Extrakt aus Traubenkernen in Mengen von 0,1 - 10 Gew.-%, bevorzugt 1 - 8 Gew.-% und besonders bevorzugt 2 - 3 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Extrakt aus Traubenkernen in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen bzw. die erfindungsgemäß verwendeten Zusammensetzungen mindestens einen Extrakt aus Schwarzen Holunderblüten (Sambucus Nigra Flower Extract). Derartige Extrakte erhöhen und/oder verbessern die Interaktion zwischen der extrazellulären Matrix und den Fibroblasten. Ein erfindungsgemäß besonders bevorzugter Extrakt aus Schwarzen Holunderblüten ist unter der Handelsbezeichnung Sambucus AO von der Firma Alpaflor/Centerchem bzw. von Permcos erhältlich. Erfindungsgemäß besonders bevorzugte kosmetische oder dermatologische Zusammensetzungen sind **dadurch** gekennzeichnet, dass sie mindestens einen Extrakt aus Schwarzen Holunderblüten in Mengen von 0,1 - 10 Gew.-%, bevorzugt 1 - 5 Gew.-% und besonders bevorzugt 2 - 3 Gew.-%, jeweils bezogen auf die gesamte Zusammensetzung, enthalten. Erfindungsgemäß besonders bevorzugte kosmetische oder dermatologische Zusammensetzungen sind **dadurch gekennzeichnet, dass** sie mindestens einen Extrakt aus Schwarzen Holunderblüten in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.ln einer weiteren bevorzugten Ausführungsform enthalten die erfindungsgemäßen bzw. die erfindungsgemäß verwendeten Zusammensetzungen mindestens ein Flavonoid oder mindestens einen Flavonoidreichen Pflanzenextrakt. In einer weiteren bevorzugten Ausführungsform enthalten die erfindungsgemäßen bzw. die erfindungsgemäß verwendeten Zusammensetzungen mindestens ein Isoflavonoid oder mindestens einen Isoflavonoid-reichen Pflanzenextrakt. Für erfindungsgemäße Flavonoid und/oder Isoflavonoid-haltige Mittel wird Schutz begehrt wird oder kann Schutz begehrt werden; Flavonoide und/oder Isoflavonoide tragen zum technischen Ziel der Erfindung und somit zur Lösung der der anmeldungsgemäßen Erfindung zugrunde liegenden technischen Aufgabe bei. Bevorzugte Flavonoide und/oder Isoflavonoide, die Mengen, in denen sie in erfindungsgemäßen Zusammensetzungen enthalten sind, sind im Prioritätsdokument DE 10 2009 026414 auf den Seiten 8 bis 9 offenbart, die dort genannten Merkmale gehören eindeutig implizit zur Beschreibung der in der eingereichten Anmeldung enthaltenen Erfindung und damit zum Offenbarungsgehalt dieser Anmeldung. Überraschenderweise zeigte die Kombination aus Isoflavonoiden und einem Reisextrakt, insbesondere einem Reisproteinhydrolysat, bevorzugt einem peptidischen Reisextrakt, einen synergistischen Effekt auf die Verringerung der Porengröße der Haut. Besonders bevorzugt sind die Kombinationen von 0,0005 bis 0,5 Gew.-% Isoflavonoiden aus Soja und 0,0001 bis 2 Gew.-% peptidischem Reisextrakt.

In einer weiteren bevorzugten Ausführungsform enthalten die erfindungsgemäßen bzw. die erfindungsgemäß verwendeten Zusammensetzungen mindestens einen Wirkstoff, der die beta-Endorphinsynthese in Keratinozyten stimuliert. Erfindungsgemäß besonders bevorzugte Stimulatoren der beta-Endorphin-Synthese sind ausgewählt aus Mischungen aus mindestens einem Extrakt aus den Blättern der Mentha piperita und mindestens einem Extrakt aus Kakaobohnen, wobei wässrige, glycolische oder wässrig-glycolische Zubereitungen dieser Extraktmischungen, die unter den Handelsbezeichnungen Caomint, Caophenol, Caobromine, Caospice und Caoorange von der Firma Solabia erhältlich sind, besonders bevorzugt sind.

Ein weiterer besonders bevorzugter Stimulator der beta-Endorphin-Synthese ist das Dipeptidderivat N-Acetyl-Tyr-Arg-hexyldecylester mit der INCI-Bezeichnung Acetyl Dipeptide-1 Cetyl Ester, das z. B. als wässrige Zubereitung unter der Handelsbezeichnung Calmosensine von der Firma Sederma erhältlich ist. Weitere bevorzugte Stimulatoren der beta-Endorphin-Synthese sind Extrakte aus Helichrysum italicum, z. B. erhältlich unter der Handelsbezeichnung Areaumat Perpetua von der Firma Codif, Extrakte aus Crithmum Maritimum, z. B. erhältlich unter den Handelsbezeichnungen Areaumat Samphira und Aroleat Samphira von der Firma Codif, Extrakte aus Lavendula stoechas, z. B. erhältlich unter der Handelsbezeichnung Areaumat Lavanda von der Firma Codif, Extrakte aus Mentha piperita, wie sie z. B. unter den Handelsbezeichnungen Authenticals of Peppermint (Solabia) und Calmiskin (Silab) erhältlich sind, Glutamylamidoethyl Indole, z. B. erhältlich unter der Handelsbezeichnung Glistin von der Firma Exsymol, ein durch mikrobielle Fermentation gewonnenes verzweigtes Polysaccharid mit Rhamnose-, Galactose- und Glucuronsäure-Einheiten mit der INCI-Bezeichnung Biosaccharide Gum-2, z. B. erhältlich unter der Handelsbezeichnung Rhamnosoft von der Firma Solabia, Extrakte aus den Samen von Tephrosia Purpurea mit der INCI-Bezeichnung Tephrosia Purpurea Seed Extract, z. B. erhältlich unter der Handelsbezeichnung Tephroline von der Firma Vincience, Mischungen aus dem Öl von Mentha arvensis-Blättern, Limonenschalenöl, Zypressenöl, Lavendelöl und Cistus Ladaniferus-Öl mit der INCI-Bezeichnung Mentha Arvensis Leaf Oil and Citrus Medica Limonum (Lemon) Peel Oil and Cupressus Sempervirens Oil and Lavandula Hybrida Oil and Cistus Ladaniferus Oil, z. B. erhältlich unter der Handelsbezeichnung V-Tonic (Gattefosse), und Hexasaccharide gemäß FR 2842201 sowie beliebige Mischungen dieser Wirkstoffe.

Erfindungsgemäß besonders bevorzugt einzusetzende kosmetische oder dermatologische Zusammensetzungen sind dadurch gekennzeichnet, dass sie mindestens einen Wirkstoff zur Stimulation der beta-Endorphin-Synthese in Gesamtmengen von 0,01 - 10 Gew.-%, bevorzugt 0,1 - 5 Gew.-% und besonders bevorzugt 1 - 3 Gew.-%, jeweils bezogen auf das Handelsprodukt, das den Wirkstoff enthält, in der gesamten erfindungsgemäßen Zusammensetzung, enthalten. Erfindungsgemäß besonders bevorzugte kosmetische oder dermatologische Zusammensetzungen sind dadurch gekennzeichnet, dass sie mindestens einen Wirkstoff zur Stimulation der beta-Endorphin-Synthese in Gesamtmengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten erfindungsgemäßen Zusammensetzung, enthalten. In einer weiteren bevorzugten Ausführungsform enthalten die erfindungsgemäßen bzw. die erfindungsgemäß verwendeten Zusammensetzungen mindestens einen Extrakt aus Haferkörnern (Avena Sativa (Oat) Kernel Extract). Ein erfindungsgemäß besonders bevorzugter Extrakt aus Haferkörnern ist unter der Handelsbezeichnung Drago Beta Glucan (02/060800) von der Firma Symrise erhältlich. Dieser Extrakt erhöht und/oder verbessert die Interaktion zwischen der extrazellulären Matrix und den Fibroblasten. Erfindungsgemäß besonders bevorzugte kosmetische oder dermatologische Zusammensetzungen sind dadurch gekennzeichnet, dass sie mindestens einen Wirkstoff, der ausgewählt ist aus Extrakten aus Haferkörnern (Avena Sativa (Oat) Kernel Extract), in einer Gesamtmenge von 0,01 - 5 Gew.-%, bevorzugt 0,1 - 3 Gew.-%, besonders bevorzugt 1 - 2 Gew.-% enthalten, jeweils bezogen auf den Gehalt an Extrakt tel quel in der gesamten Zusammensetzung. Erfindungsgemäß besonders bevorzugte kosmetische oder dermatologische Zusammensetzungen sind dadurch gekennzeichnet, dass sie mindestens einen Wirkstoff, der ausgewählt ist aus Extrakten aus Haferkörnern (Avena Sativa (Oat) Kernel Extract), in einer Gesamtmenge von 0,00001 - 1 Gew.-%, bevorzugt 0,0001 - 0,1 Gew.-%, besonders bevorzugt 0,001 - 0,05 Gew.-% enthalten, jeweils bezogen auf den Gehalt an Aktivsubstanz in der gesamten Zusammensetzung.

In einer weiteren bevorzugten Ausführungsform enthalten die erfindungsgemäßen bzw. die erfindungsgemäß verwendeten Zusammensetzungen mindestens ein Produkt, das durch Fermentation von gezuckertem Schwarztee mit den zwei symbiotischen Mikroorganismen Saccharomyces und Acetobacter ylinum gewonnen wird, als Kombucha bezeichnet wird und die INCI-Bezeichnung Saccharomyces/ Xylinum/Black Tea Ferment trägt. Derartige Produkte erhöhen und/oder verbessern die Interaktion zwischen der extrazellulären Matrix und den Fibroblasten. Ein besonders bevorzugtes Produkt ist unter dem Handelsnamen Kombuchka von der Firma Sederma erhältlich (INCI-Bezeichnung: Saccharomyces/ Xylinum/Black Tea Ferment, Glycerin, Hydroxyethylcellulose). Erfindungsgemäß besonders bevorzugte kosmetische oder dermatologische Zusammensetzungen sind dadurch gekennzeichnet, dass sie mindestens einen Wirkstoff, der ausgewählt ist aus Produkten, die durch Fermentation von gezuckertem Schwarztee mit den zwei symbiotischen Mikroorganismen Saccharomyces und Xylinum gewonnen werden und die INCI-Bezeichnung Saccharomyces/Xylinum/ Black Tea Ferment tragen, in einer Gesamtmenge von 0,01 - 5 Gew.-%, bevorzugt 0,1 - 3 Gew.-%, besonders bevorzugt 1 - 2 Gew.-% enthalten, jeweils bezogen auf den Gehalt an Produkt tel quel in der gesamten Zusammensetzung. Erfindungsgemäß besonders bevorzugte kosmetische oder dermatologische Zusammensetzungen sind dadurch gekennzeichnet, dass sie mindestens einen Wirkstoff, der ausgewählt ist aus Produkten, die durch Fermentation von gezuckertem Schwarztee mit den zwei symbiotischen Mikroorganismen Saccharomyces und Acetobacter Xylinum gewonnen werden und die INCI-Bezeichnung Saccharomyces/Xylinum/Black Tea Ferment tragen, in einer Gesamtmenge von 0,00001 - 1 Gew.-%, bevorzugt 0,0001 - 0,1 Gew.-%, besonders bevorzugt 0,001 - 0,05 Gew.-% enthalten, jeweils bezogen auf den Gehalt an Aktivsubstanz in der gesamten Zusammensetzung Gemäß einer weiteren bevorzugten Ausführungsform wird der Reisextrakt, insbesondere das Reisproteinhydrolysat, bevorzugt der weiter oben definierte peptidische Reisextrakt, in Kombination mit wenigstens einem weiteren, vorzugsweise mehreren, Inhaltsstoffe eingesetzt wird, ausgewählt aus mindestens einem sebumregulierenden Wirkstoff, mindestens einem partikelförmigen, sebumsorbierenden Wirkstoff und mindestens einem adstringierenden Wirkstoff, Absorptionsmitteln, antimikrobiellen Stoffen, Stoffen biologischen Ursprungs, Chelatbildnern, Emollientien, Emulgatoren/Dispergatoren, Emulsionsstabilisatoren, Feuchtigkeitsspendern, Filmbildnern, Konservierungsstoffen, hautkühlenden Wirkstoffen, Tensiden/waschaktiven Substanzen, Treibgasen, Trübungsmitteln, mindestens ein Vitamin, Provitamin oder eine als Vitaminvorstufe bezeichnete Verbindung aus den Vitamingruppen A, B, H und K und den Estern der vorgenannten Substanzen, Hydrogelbildnern, Lösungsmitteln, mindestens einem alkyl- oder hydroxyalkylsubstituierten Harnstoff der allgemeinen Formel (A), 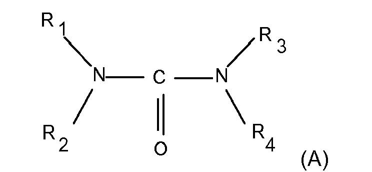 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt, weiterhin ausgewählt aus mindestens einem weiteren kosmetischen Wirkstoff, der ausgewählt ist aus Monomeren, Oligomeren und Polymeren von Aminosäuren, N-C₂-C₂₄-Acylaminosäuren und/oder den Estern und/oder den physiologisch verträglichen Metallsalzen dieser Substanzen, sowie Mischungen dieser Wirkstoffe, mindestens einem DNA-Oligonucleotid oder mindestens einem RNA-Oligonucleotid und mindestens einer natürlichen Betainverbindung.

In einer bevorzugten Ausführungsform enthalten die erfindungsgemäßen bzw. die erfindungsgemäß verwendeten Zusammensetzungen zur Unterstützung der Wirkung des erfindungsgemäß verwendeten Reisextrakts mindestens einen sebumregulierenden und/oder sebumsorbierenden Wirkstoff. Für erfindungsgemäße sebumsorbierende und/oder sebumregulierende Wirkstoff haltige Mittel wird Schutz begehrt wird oder kann Schutz begehrt werden; Sebumregulierende und/oder sorbierenden Wirkstoffe tragen zum technischen Ziel der Erfindung und somit zur Lösung der der anmeldungsgemäßen Erfindung zugrunde liegenden technischen Aufgabe bei. Bevorzugte sebumregulierende und/oder sebumsorbierende Wirkstoffe, die Mengen, in denen sie in erfindungsgemäßen Zusammensetzungen enthalten sind sind im Prioritätsdokument DE 10 2009 026414 auf den Seiten 13 bis 16 offenbart, die dort genannten Merkmale gehören eindeutig implizit zur Beschreibung der in der eingereichten Anmeldung enthaltenen Erfindung und damit zum Offenbarungsgehalt dieser Anmeldung. Zur Unterstützung des erfindungsgemäß verwendeten Reisextrakts enthalten die erfindungsgemäßen Hautbehandlungsmittel bevorzugt weiterhin mindestens einen adstringierenden Wirkstoff, der zu einer temporären Verengung der Hautporen führt, beispielsweise Tannine bzw. Gerbstoffe bzw. allgemein Polyphenole, weiterhin Aluminiumsalze, wie sie auch als Antitranspirant-Wirkstoffe Verwendung finden. Diese können allerdings in Verbindung mit dem erfindungsgemäß verwendeten Reisextrakt in geringeren Konzentrationen als üblich eingesetzt werden. Überraschenderweise zeigte die Kombination aus sebumregulierendem Wirkstoff, und dem Reisextrakt, insbesondere dem Reisproteinhydrolysat, bevorzugt einem peptidischen Reisextrakt, einen synergistischen Effekt auf die Verringerung der Porengröße der Haut.

Besonders bevorzugt werden Kombinationen von 0,001 bis 5 Gew.-% 10-Hydroxydecansäure und 0,0001 bis 2 Gew.-% peptidischem Reisextrakt, 0,001 bis 5 Gew.-% Sebacinsäure und 0,0001 bis 25 Gew.-% peptidischem Reisextrakt, 0,001 bis 5 Gew.-% Azelainsäure und/oder den Estern der Azelainsäure und 0,0001 bis 2 Gew.-% peptidischem Reisextrakt zur Verringerung der Porengröße eingesetzt.

In einer weiteren bevorzugten Ausführungsform enthalten die erfindungsgemäßen bzw. die erfindungsgemäß verwendeten Zusammensetzungen mindestens einen Desoxyzucker oder ein mindestens einen Desoxyzucker-Baustein enthaltendes Polysaccharid.

Erfindungsgemäß bevorzugte Desoxyzucker sind ausgewählt aus Rhamnose und Fucose. Ein erfindungsgemäß besonders bevorzugtes, Desoxyzucker-Bausteine enthaltendes Polysaccharid ist das Handelsprodukt Fucogel^{®} von Solabia mit der INCI-Bezeichnung Biosaccharide Gum-1. Ein weiteres erfindungsgemäß besonders bevorzugtes, Desoxyzucker-Bausteine enthaltendes Polysaccharid ist das Handelsprodukt Rhamnosoft^{®} von Solabia mit der INCI-Bezeichnung Biosaccharide Gum-2. Ein erfindungsgemäß besonders bevorzugtes, Desoxyzucker-Bausteine enthaltendes Polysaccharid ist das Handelsprodukt Fucogenol^{®} von Solabia mit der INCI-Bezeichnung Biosaccharide Gum-3. Ein erfindungsgemäß besonders bevorzugtes, Desoxyzucker-Bausteine enthaltendes Polysaccharid ist das Handelsprodukt Glycofilm^{®} von Solabia mit der INCI-Bezeichnung Biosaccharide Gum-4. Erfindungsgemäß bevorzugt sind weiterhin Mischungen der vorgenannten, mindestens einen Desoxyzucker-Baustein enthaltenden Polysaccharide, beispielsweise die Mischung aus Biosaccharide Gum-2 und Biosaccharide Gum-3, erhältlich als Handelsprodukt Elastinol plus^{®} von Solabia.

Erfindungsgemäß besonders bevorzugte kosmetische oder dermatologische Zusammensetzungen sind dadurch gekennzeichnet, dass sie mindestens einen Desoxyzucker und/ oder mindestens ein Desoxyzucker-Bausteine enthaltendes Polysaccharid in Gesamtmengen von 0,001 bis 5 Gew.-%, bevorzugt 0,01 bis 2 Gew.-% und besonders bevorzugt 0,1 bis 1 Gew.-%, jeweils bezogen auf die gesamte erfindungsgemäße Zusammensetzung, enthalten.

In einer bevorzugten Ausführungsform enthalten die erfindungsgemäßen bzw. die erfindungsgemäß verwendeten Zusammensetzungen mindestens einen weiteren kosmetischen Wirkstoff, der ausgewählt ist aus Monomeren, Oligomeren und Polymeren von Aminosäuren, N-C₂-C₂₄-Acylaminosäuren und/oder den Estern und/oder den physiologisch verträglichen Metallsalzen dieser Substanzen, sowie Mischungen dieser Wirkstoffe.

Für erfindungsgemäße Mittel, die mindestens einen weiteren kosmetischen Wirkstoff, der ausgewählt ist aus Monomeren, Oligomeren und Polymeren von Aminosäuren, N-C₂-C₂₄-Acyl-aminosäuren und/oder den Estern und/oder den physiologisch verträglichen Metallsalzen dieser Substanzen, sowie Mischungen dieser Wirkstoffe enthalten, wird Schutz begehrt wird oder kann Schutz begehrt werden; Diese Substanzen tragen zum technischen Ziel der Erfindung und somit zur Lösung der der anmeldungsgemäßen Erfindung zugrunde liegenden technischen Aufgabe bei. Bevorzugte Substanzen, die Mengen, in denen sie in erfindungsgemäßen Zusammensetzungen enthalten sind sind im Prioritätsdokument DE 10 2009 026414 auf den Seiten 18 bis 24 offenbart, die dort genannten Merkmale gehören eindeutig implizit zur Beschreibung der in der eingereichten Anmeldung enthaltenen Erfindung und damit zum Offenbarungsgehalt dieser Anmeldung. Erfindungsgemäß ganz besonders bevorzugt ist die Kombination von Reisextrakt, bevorzugt peptidischem Reisextrakt, insbesondere Reisproteinhydrolysat und einer Mischung aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich ist. Besonders bevorzugt ist weiterhin die Kombination von Reisextrakt, bevorzugt peptidischem Reisextrakt, insbesondere Reisproteinhydrolysat mit Sojaproteinhydrolysaten, besonders bevorzugt Sojaproteinhydrolysaten mit einem mittleren Molekulargewicht im Bereich von 1200 - 1800 Dalton, bevorzugt im Bereich von 1400 - 1700 Dalton, z. B. unter dem Handelsnamen Ridulisse C^{®} von der Firma Silab erhältlich, und Sojaproteinhydrolysate mit einem mittleren Molekulargewicht im Bereich von 600 - 1000 Dalton, bevorzugt 800 Dalton, z. B. unter dem Handelsnamen Phytokine^{®} von Coletica erhältlich. mit Kokosfettsäuren N-acylierten und/oder veresterten Sojaproteinhydrolysaten in Form ihrer Alkalimetallsalze.

In einer weiteren bevorzugten Ausführungsform enthalten die erfindungsgemäßen bzw. die erfindungsgemäß verwendeten Zusammensetzungen mindestens eine natürliche Betainverbindung. Erfindungsgemäße natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Carnitin (Me₃N⁺-CH₂-CHOH-CH₂-COO⁻) und Betain (Me₃N⁺-CH₂-COO jeweils mit Me = Methyl und X = C-C-Einfachbindung (im Falle des Betains) oder X = -CHOH-CH₂- (im Falle des Carnitins). Erfindungsgemäß besonders bevorzugte Zusammensetzungen sind dadurch gekennzeichnet, dass sie mindestens eine natürliche Betainverbindung in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten. Es hat sich überraschenderweise gezeigt, daß sich Betain (Trimethylammoniumacetat) besonders gut in die erfindungsgemäß zu verwendenden Zusammensetzungen einarbeiten läßt und die erfindungsgemäße Wirkstoffkombination unterstützt, indem die hautberuhigenden und -pflegenden Effekte weiter verstärkt werden. Eine weitere erfindungsgemäß besonders bevorzugte Zusammensetzung ist daher dadurch gekennzeichnet, dass sie bezogen auf ihr Gewicht 0,5 bis 5 Gew.-%, vorzugsweise 1,0 bis 4 Gew.-%, besonders bevorzugt 1,5 bis 3 Gew.-% und insbesondere 1,75 bis 2,5 Gew.-% Trimethylammoniumacetat (H₃C)₃N⁺CH₂COO⁻ enthält.

Für erfindungsgemäße antimikrobielle Wirkstoffe bzw. Konservierungsmittel haltige Mittel wird Schutz begehrt wird oder kann Schutz begehrt werden; antimikrobielle Wirkstoffe bzw. Konservierungsmittel tragen zum technischen Ziel der Erfindung und somit zur Lösung der der anmeldungsgemäßen Erfindung zugrunde liegenden technischen Aufgabe bei. Bevorzugte antimikrobielle Wirkstoffe bzw. Konservierungsmittel, die Mengen, in denen sie in erfindungsgemäßen Zusammensetzungen enthalten sind sind im Prioritätsdokument DE 10 2009 026414 auf den Seiten 25 bis 27 offenbart, die dort genannten Merkmale gehören eindeutig implizit zur Beschreibung der in der eingereichten Anmeldung enthaltenen Erfindung und damit zum Offenbarungsgehalt dieser Anmeldung.

Weitere bevorzugte erfindungsgemäße Zusammensetzungen sind dadurch gekennzeichnet, dass zur Verbesserung der Hautfeuchtigkeit unter Verzicht auf komedogene Substanzen mindestens ein alkyl- oder hydroxyalkylsubstituierten Harnstoff der allgemeinen Formel (A, s.o.) enthalten ist, in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt. Weitere bevorzugte erfindungsgemäße Zusammensetzungen sind **dadurch gekennzeichnet, dass** der mindestens eine alkyl- oder hydroxyalkylsubstituierte Harnstoff der allgemeinen Formel (A) ausgewählt ist aus Verbindungen, in denen R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-HydroxyalkylGruppe, die mit 1 bis 5 Hydroxylgruppen oder C₁-C₄-Hydroxyalkyl-Gruppen substituiert ist, stehen, mit der Maßgabe, dass mindestens einer der Reste R₁ und R₂ und gleichzeitig mindestens einer der Reste R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt. Weitere bevorzugte erfindungsgemäße Zusammensetzungen sind **dadurch gekennzeichnet, dass** der mindestens eine alkyl- oder hydroxyalkylsubstituierte Harnstoff der allgemeinen Formel (A) ausgewählt ist aus Bis-N,N'- (2-hydroxyethyl)harnstoff. Erfindungsgemäß besonders bevorzugte Zusammensetzungen sind **dadurch gekennzeichnet, dass** sie mindestens einen alkyl- oder hydroxyalkylsubstituierten Harnstoff gemäß Formel (A), insbesondere N,N'-Bis-(2-hydroxyethyl)harnstoff, in einer Gesamtmenge von 0,01 - 50 Gew.-%, bevorzugt 0,1 - 20 Gew.-%, besonders bevorzugt 1 - 10 Gew.-% und außerordentlich bevorzugt 2 - 5 Gew.-%, bezogen auf die gesamte Zusammensetzung, enthalten. N,N'-Bis-(2-hydroxyethyl)harnstoff, ein feuchtigkeitsspendender und hautweichmachender Wirkstoff, der hervorragend mit der erfindungsgemäßen Wirkstoffkombination harmoniert und seine Wirkung mit diesem schneller und nachhaltiger entfaltet,bei Raumtemperatur ein kristalliner Feststoff, ist beispielsweise als Handelsprodukt Hydrovance der National Starch and Chemical Company als ca. 45 - 55 %ige wässrige Lösung mit einem Gehalt an Harnstoff und Ammoniumlactat erhältlich. Die optionalen teilchenförmigen Feststoffe können sowohl einzeln als auch im Gemisch eingesetzt werden. Die Gesamtmenge der optionalen teilchenförmigen Feststoffe beträgt bevorzugt 0,1 - 30 Gew.-%, besonders bevorzugt 0,5 - 15 Gew.-%, außerordentlich bevorzugt 1,0 - 10 Gew.-%, oder auch 2 - 4 Gew.-%, jeweils bezogen auf das Gesamtgewicht der erfindungsgemäßen Zusammensetzung.

Für erfindungsgemäße chelat-bzw. komplexbildnerhaltige und/oder trübungsmittelhaltige und/oder treibgashaltige und/oder tensidhaltige und/oder hautkühlende Wirkstoff-haltige und/oder feuchtigkeitsspenderhaltige und/oder filmbildnerhaltige und/oder emollientienhaltige und/oder emulsionsstabilisatorhaltige und/oder Emulgator- bzw. Dispergatorhaltige und/oder Stoffe biologischen Ursprungs haltige Mittel wird Schutz begehrt wird oder kann Schutz begehrt werden; Diese Substanzen tragen zum technischen Ziel der Erfindung und somit zur Lösung der der anmeldungsgemäßen Erfindung zugrunde liegenden technischen Aufgabe bei. Bevorzugte Substanzen, die Mengen, in denen sie in erfindungsgemäßen Zusammensetzungen enthalten sind sind im Prioritätsdokument DE 10 2009 026414 auf den Seiten 33 bis 51 offenbart, die dort genannten Merkmale gehören eindeutig implizit zur Beschreibung der in der eingereichten Anmeldung enthaltenen Erfindung und damit zum Offenbarungsgehalt dieser Anmeldung. Bevorzugte Chelatbildner sind im Prioritätsdokument DE 10 2009 026414 auf den Seiten 48 bis 51, bevorzugte Tenside auf den Seiten 41 bis 48 (ebenda) offenbart, bevorzugte Emollientien sind auf den Seiten 29 bis 33 (ebenda), bevorzugte Emulgatoren /Dispergatoren auf den Seiten 33 bis 39 (ebenda) offenbart.

Weiterhin können enthalten sein Antischaummittel, (z.B. um Schaum während der Herstellung zu beseitigen oder um die Neigung von Fertigprodukten zur überschießenden Schaumbildung zu verringern, Bindemittel (die z.B. den Zusammenhalt pulver- und puderhaltiger kosmetischer Produkte gewährleisten), Bleichmittel (die z.B. dazu dienen, den Farbton des Haares oder der Haut aufzuhellen), Farbstoffe (um eine Produktfärbung zu erzeugen), Parfums und/oder Parfümöle (um der Zusammensetzung einen angenehmen Duft zu verleihen), pH-Wert-Regler und Puffersubstanzen (um einen gewünschten pH-Wert einzustellen bzw. zu stabilisieren), Vergällungsmittel (die zugesetzt werden, um die Zusammensetzungen ungenießbar zu machen), Viskositätsregler (um die Zähflüssigkeit eines Produkts zu erhöhen oder auch zu verringern). Vorgenannte Inhaltsstoffe können gemäß weiterer bevorzugter Ausführungsformen als einziger Zusatz oder in beliebigen Kombinationen in den erfindungsgemäß zu verwendenden Zusammensetzungen enthalten sein. Es sei an dieser Stelle darauf hingewiesen, dass sich alle Angabe Gew.-%, sofern es nicht anders angegeben ist, jeweils auf das gesamte Mittel bezieht. Der Gehalt an Wasser in bevorzugten erfindungsgemäßen Mitteln ist im Prioritätsdokument DE 10 2009 026414 auf den Seiten 51 bis 52 offenbart, die dort genannten Merkmale gehören eindeutig implizit zur Beschreibung der in der eingereichten Anmeldung enthaltenen Erfindung und damit zum Offenbarungsgehalt dieser Anmeldung. Bevorzugte Wirkstoffe gegen Akne und unreine Haut sind ausgewählt aus sogenannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime, die zu einer gesunden Hautmikroflora gehören. Explizit sind hier die Wirkstoffe, die in den Offenlegungsschriften DE 10333245 und DE 10 2004 011 968 als präbiotisch wirksam offenbart sind, mit einbezogen; dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp., Panax sp., Lamium album* oder Ribes *nigrum* sowie Mischungen dieser Substanzen. Besonders bevorzugt ist eine Kombination von Extrakten von Panax, Ribes und Pinus zusammen mit Reisextrakt, bevorzugt peptidischen Reisextrakt, insbesondere Reisproteinhydrolysat.

In einer weiteren bevorzugten Ausführungsform liegen die erfindungsgemäßen Mittel in partikulärer Form vor. Für erfindungsgemäße partikuläre Mittel wird Schutz begehrt wird oder kann Schutz begehrt werden; Partikuläre Ausführungsformen tragen zum technischen Ziel der Erfindung und somit zur Lösung der der anmeldungsgemäßen Erfindung zugrunde liegenden technischen Aufgabe bei. Bevorzugte partikuläre Ausführungsformen, die Art und Mengen der erfindungsgemäßen Zusammensetzungen, sind im Prioritätsdokument DE 10 2009 026414 auf den Seiten 52 bis 53 offenbart, die dort genannten Merkmale gehören eindeutig implizit zur Beschreibung der in der eingereichten Anmeldung enthaltenen Erfindung und damit zum Offenbarungsgehalt dieser Anmeldung.

Gemäß einer anderern besonders bevorzugten Ausführungsform liegt die erfindungsgemäße Zusammensetzung in flüssiger Form vor. Zum Erreichen einer flüssigen Konsistenz kann der Einsatz sowohl flüssiger organischer Lösungsmittel, wie auch der von Wasser angezeigt sein. Bevorzugte erfindungsgemäße Mittel enthalten daher gegebenenfalls mindestens ein Lösungsmittel. Für erfindungsgemäße Lösungsmittelhaltige Mittel wird Schutz begehrt wird oder kann Schutz begehrt werden; Lösungsmittel tragen zum technischen Ziel der Erfindung und somit zur Lösung der der anmeldungsgemäßen Erfindung zugrunde liegenden technischen Aufgabe bei. Bevorzugte Lösungsmittel, die Mengen, in denen sie in erfindungsgemäßen Zusammensetzungen enthalten sind sind im Prioritätsdokument DE 10 2009 026414 auf den Seiten 53 bis 54 offenbart, die dort genannten Merkmale gehören eindeutig implizit zur Beschreibung der in der eingereichten Anmeldung enthaltenen Erfindung und damit zum Offenbarungsgehalt dieser Anmeldung.

In einer weiteren bevorzugten Ausführungsform enthalten die erfindungsgemäßen bzw. die erfindungsgemäß verwendeten Zusammensetzungen mindestens ein Vitamin, Provitamin oder eine als Vitaminvorstufe bezeichnete Verbindung aus den Vitamingruppen A, B, H und K und den Estern der vorgenannten Substanzen. Für erfindungsgemäße Vitaminhaltige Mittel wird Schutz begehrt wird oder kann Schutz begehrt werden; Vitamine, Provitamine oder als Vitaminvorstufe bezeichnete Verbindungen tragen zum technischen Ziel der Erfindung und somit zur Lösung der der anmeldungsgemäßen Erfindung zugrunde liegenden technischen Aufgabe bei. Bevorzugte Vitamine, Provitamine oder als Vitaminvorstufe bezeichnete Verbindungen, die Mengen, in denen sie in erfindungsgemäßen Zusammensetzungen enthalten sind sind im Prioritätsdokument DE 10 2009 026414 auf den Seiten 54 bis 58 offenbart, die dort genannten Merkmale gehören eindeutig implizit zur Beschreibung der in der eingereichten Anmeldung enthaltenen Erfindung und damit zum Offenbarungsgehalt dieser Anmeldung.

In einer weiteren bevorzugten Ausführungsform enthalten die erfindungsgemäßen bzw. die erfindungsgemäß verwendeten Zusammensetzungen neben der erfindungsgemäßen Wirkstoffkombination mindestens eine α-Hydroxycarbonsäure, α-Ketocarbonsäure oder β-Hydroxycarbonsäure oder deren Ester-, Lacton- oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Erfindungsgemäß besonders bevorzugte Zusammensetzungen sind dadurch gekennzeichnet, dass sie neben der erfindungsgemäßen Wirkstoffkombination mindestens eine α-Hydroxycarbonsäure, α-Ketocarbonsäure und/oder β-Hydroxycarbonsäure oder ein Derivat, insbesondere einen Ester, ein Lacton oder ein Salz hiervon, insbesondere Salicylsäure, in einer Gesamtmenge von 0,01 - 10 Gew.-%, bevorzugt 0,1 - 5 Gew.-%, besonders bevorzugt 0,5 - 1 - 2 Gew.-%, jeweils bezogen auf die gesamte Zusammensetzung, enthalten.

In einer weiteren bevorzugten Ausführungsform enthalten die erfindungsgemäßen bzw. die erfindungsgemäß verwendeten Zusammensetzungen mindestens ein Polyphenol oder einen Polyphenol-reichen Pflanzenextrakt und/oder Ectoin und/oder Kreatin und/oder Ursolsäure und/oder mindestens ein natürlich vorkommendes Xanthin-Derivat. Unter Polyphenolen sind erfindungsgemäß aromatische Verbindungen zu verstehen, die mindestens zwei phenolische Hydroxy-Gruppen im Molekül enthalten. Für erfindungsgemäße polyphenolhaltige und/oder Ectoin-und/oder Kreatin- und/oder Ursolsäure und/oder Xanthin-Derviathaltige Mittel wird Schutz begehrt wird oder kann Schutz begehrt werden; Polyphenole und/oder Ectoin und/oder Kreatin und/oder Ursolsäure und/oder Xanthin-Derviate tragen zum technischen Ziel der Erfindung und somit zur Lösung der der anmeldungsgemäßen Erfindung zugrunde liegenden technischen Aufgabe bei. Bevorzugte Polyphenole sowie Ectoin, Kreatin Ursolsäure und bevorzugte Xanthin-Derviate, die Mengen, in denen sie in erfindungsgemäßen Zusammensetzungen enthalten sind sind im Prioritätsdokument DE 102009026414 auf den Seiten 58 bis 61 offenbart, die dort genannten Merkmale gehören eindeutig implizit zur Beschreibung der in der eingereichten Anmeldung enthaltenen Erfindung und damit zum Offenbarungsgehalt dieser Anmeldung.

In einer weiteren bevorzugten Ausführungsform enthalten die erfindungsgemäßen bzw. die erfindungsgemäß verwendeten Zusammensetzungen mindestens einen Olivenblattextrakt (Olea Europaea (Olive) Leaf Extract). Ein erfindungsgemäß besonders bevorzugter Olivenblattextrakt ist unter der Handelsbezeichnung Oleanoline DPG von der Firma Vincience erhältlich. Ein weiterer erfindungsgemäß besonders bevorzugter Olivenblattextrakt ist unter der Handelsbezeichnung Olea europ Fol extr. S. sicc. von der Firma Fruitarom erhältlich.

Erfindungsgemäß besonders bevorzugte Zusammensetzungen sind dadurch gekennzeichnet, dass sie mindestens einen Olivenblattextrakt in einer Gesamtmenge von 0,01 bis 5 Gew.-%, bevorzugt 0,1 bis 3 Gew.-% und besonders bevorzugt 0,5 bis 1 - 2 Gew.-%, jeweils bezogen auf den Extrakt als Handelsprodukt tel quel in der gesamten erfindungsgemäßen Zusammensetzung, enthalten. Olivenblattextrakte können einen hohen Gehalt an Oleanolsäure und/oder Oleanol aufweisen. In einer weiteren bevorzugten Ausführungsform enthalten die erfindungsgemäßen bzw. die erfindungsgemäß verwendeten Zusammensetzungen Oleanolsäure, Oleanol und/oder Oleuropein. Erfindungsgemäß besonders bevorzugte Zusammensetzungen sind dadurch gekennzeichnet, dass sie Oleanolsäure, Oleanol und/oder Oleuropein in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Zusammensetzung, enthalten.

In einer weiteren bevorzugten Ausführungsform enthalten die erfindungsgemäßen bzw. die erfindungsgemäß verwendeten Zusammensetzungen mindestens ein Derivat von methyliertem Silanol, vorzugsweise mindestens einen Ester von methyliertem Silanol. Für erfindungsgemäße Derivate von methyliertem Silanol haltige Mittel wird Schutz begehrt wird oder kann Schutz begehrt werden; Derivate von methyliertem Silanol tragen zum technischen Ziel der Erfindung und somit zur Lösung der der anmeldungsgemäßen Erfindung zugrunde liegenden technischen Aufgabe bei. Bevorzugte Derivate von methyliertem Silanol, die Mengen, in denen sie in erfindungsgemäßen Zusammensetzungen enthalten sind sind im Prioritätsdokument DE 10 2009 026414 auf der Seite 61 offenbart, die dort genannten Merkmale gehören eindeutig implizit zur Beschreibung der in der eingereichten Anmeldung enthaltenen Erfindung und damit zum Offenbarungsgehalt dieser Anmeldung.

Gemäß einer weiteren besonders bevorzugten Ausführungsform wird der Reisextrakt, insbesondere das Reisproteinhydrolysat, bevorzugt der peptidische Reisextrakt in Kombination mit einem Antioxidans ausgewählt aus substituierten oder nicht substituierten 2,2-Dialkylchroman-Derivaten, bevorzugt ausgewählt aus Tocopherolen, Tocopherylacetat oder 6,7-disubstituiertes 2,2-Dialkylchroman oder -chromen, besonders bevorzugt ausgewählt aus Tocopherylacetat und Dimethylmethoxy Chromanol, zur Verringerung der Porengröße verwendet. Bekannte 2,2-Dialkylchroman-Derivate sind vor allem Tocopherole und Tocotrienole. Die Tocopherole sind in 2-Position mit einem Methylrest und mit einem gesättigten 4',8',12'-Trimethyltridecyl-Rest substituiert und weisen in 5-, 7- und 8-Position entweder ein Wasserstoffatom oder eine Methylgruppe auf (α-, β-, γ-, δ-, ζ₂- und η-Tocopherole). Die Tocotrienole, auch als ε-Tocopherole bezeichnet, unterscheiden sich von den vorgenannten Tocopherolen dadurch, dass in 2-Position an Stelle des gesättigten 4',8',12'-Trimethyltridecyl-Restes ein dreifach ungesättigter 4',8',12'-Trimethyltridecatrienyl-Rest aus drei Isoprenyl-Einheiten vorhanden ist. Neben ihrer Anwendung als Vitamin E wirken Tocopherole als Antioxidantien in Fetten und Ölen. Darüber hinaus besitzen Tocopherole eine Vielzahl von günstigen physiologischen Eigenschaften, z. B. Reduzierung von Muskelschäden, die auf oxidativen Streß während körperlicher Höchstleistung zurückzuführen sind, Verminderung des Risikos der Kataraktbildung, Verminderung des oxidativen Stresses bei Rauchern sowie anticarcinogene Effekte. Kosmetisch zeigen sie eine protektive Wirkung gegen Hautschäden und Hautalterung und schützen das Haar vor Witterungseinflüssen. Neben den erwähnten Tocopherolen und Tocotrienolen sind weitere 2,2-Dialkylchroman- Derivate als kosmetische Wirkstoffe bekannt. Die Offenlegungsschrift EP 1 174 140 A1 offenbart Chromanolglycoside als Wirkstoff, der effektiv aktiven Sauerstoff und freie Radikale eliminiert. Explizit offenbart ist 2,5,7,8-Tetramethylchroman-6-ol, das in 2-Position mit einem Glucopyranosylmethyl-, Galactopyranosylmethyl-, Fructofuranosylmethyl- oder Mannopyranosylmethyl-Rest substituiert ist. Die Patentschrift US 5,811,083 offenbart das Amid aus 3,4-Dihydro-6-hydroxy-2,5,7,8-tetramethyl-1-benzopyran-2-carbonsäure (Trolox C) und Cysteamin als Antioxidans gegen Lipidperoxidation.

Die Offenlegungsschrift EP 655 239 A1 offenbart die Verwendung von 2,2-Dimethylchromanen und -chromenen, die in 6- und 7-Position mit OH-, CH₃O- oder CF₃CH₂O-Gruppen substituiert sein können, als Antioxidantien in Doxorubicin-haltigen Liposomen. Daran anknüpfend, offenbart die Offenlegungsschrift EP 1 002 533 A1 die Verwendung der genannten 2,2-Dimethylchromane und - chromene als Antioxidantien in Zusammensetzungen zur topischen oder oralen Verabreichung. Überraschend und für den Fachmann nicht vorhersehbar wurde nun gefunden, dass ausgewählte 6,7-disubstituierte 2,2-Dialkylchromane und -chromene in topischen kosmetischen und pharmazeutischen Zusammensetzungen die positive Wirkung des erfindungsgemäßen Reisextrakts auf die Haut steigern. Insbesondere bevorzugt enthalten die erfindungsgemäß zu verwendenden Zusammensetzungen mindestens ein 6,7-disubstituiertes 2,2-Dialkylchroman oder - chromen der allgemeinen Formel (I) oder (II), 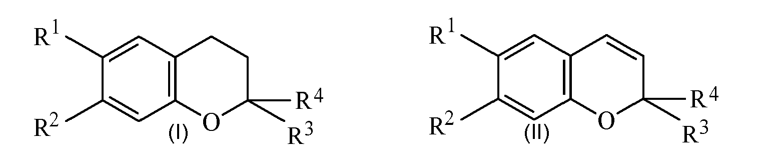 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, und mindestens ein weiteres Antioxidans, ausgewählt aus Flavonoiden, Polyphenolen, Catechinen, Ubichinonen. In einer weiteren bevorzugten Ausführungsform enthalten die erfindungsgemäßen bzw. die erfindungsgemäß verwendeten Zusammensetzungen mindestens ein Ubichinon oder ein Ubichinol oder deren Derivate. Ubichinole sind die reduzierte Form der Ubichinone. Besonders bevorzugt ist das Ubichinon, welches auch bekannt ist als Coenzym Q10. Erfindungsgemäß besonders bevorzugte Zusammensetzungen sind **dadurch gekennzeichnet, dass** sie mindestens ein Ubichinon, Ubichinol oder ein Derivat hiervon in einer Gesamtmenge von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, enthalten. Erfindungsgemäß besonders bevorzugte zu verwendende Zusammensetzungen sind **dadurch gekennzeichnet, dass** sie mindestens eine Substanz, ausgewählt substituierten oder nicht substituierten 2,2-Dialkylchroman-Derivaten, in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

Überraschenderweise zeigte die Kombination aus Tocopherylacetat und dem Reisextrakt, insbesondere dem Reisproteinhydrolysat, bevorzugt einem peptidischen Reisextrakt, einen synergistischen Effekt auf die Verringerung der Porengröße der Haut. Besonders bevorzugt sind die Kombinationen von 0,1 bis 3 Gew.-% Tocopherylacetat und 0,0001 bis 2 Gew.-% peptidischem Reisextrakt. Weiterhin bevorzugt ist die Kombination von Dimethylmethoxy Chromanol und dem Reisextrakt, insbesondere dem Reisproteinhydrolysat, bevorzugt einem peptidischen Reisextrakt, einen synergistischen Effekt auf die Verringerung der Porengröße der Haut. Besonders bevorzugt sind die Kombinationen von 0,1 bis 3 Gew.-% Dimethylmethoxy Chromanol und 0,0001 bis 2 Gew.-% peptidischem Reisextrakt.

Ein weiterer Gegenstand der vorliegenden Erfindung sind kosmetische Zusammensetzungen, enthaltend mindestens einen Reisextrakt und mindestens ein Salz von C₁₂₋₂₀-Alkylphosphat, insbesondere ein Salz von Cetylphosphat. Diese Salze von C12 bis C20 -Alkylphophaten, insbesondere die Salze von Cetylphosphat, eignen sich in Kombination mit Reisextrakt überraschender Weise besonders gut als anionische Öl-in-Wasser-Emulgatoren. Erfindungsgemäß bevorzugte Salze von C₁₂₋₂₀-Alkylphosphaten, die besonders vorteilhaft als anionischer Öl-in-Wasser-Emulgator eingesetzt werden können, sind ausgewählt aus den Mono-und/oder Diestern von Phosphorsäure mit Laurylalkohol, Tridecylalkohol, Isotridecylalkohol, Myristylalkohol, Pentadecylalkohol, Cetylalkohol, Palmitylalkohol, Isocetylalkohol, Isostearylalkohol, Stearylalkohol, Oleylalkohol, Elaidylalkohol, Linoleylalkohol, Linolenylalkohol, Nonadecylalkohol, Arachylalkohol, Gadoleylalkohol oder Arachidonalkohol, die als Alkali-, Erdalkali-, Ammonium-, Alkylammonium-, Alkanolammonium- oder Glucammoniumsalz vorliegen. Bevorzugt sind die Kaliumsalze der genannten Phosphorsäuremonoester. Besonders bevorzugt ist Dikaliummonocetylphosphat. Bevorzugt bei den Diestern sind die Kaliumsalze der genannten Phosphorsäurediester. Besonders bevorzugt ist Kaliumdicetyl-phosphat. Besonders bevorzugt sind Mischungen aus Mono-C₁₂₋₂₀-Alkylphosphaten und Di-C₁₂₋₂₀-Alkylphosphaten. Außerordentlich bevorzugt sind Mischungen aus Dikaliummonocetylphosphat und Kaliumdicetylphosphat. Weiterhin können alle Inhaltsstoffe, die in Kombination mit dem Reisextrakt verwendet werden sollen, bevorzugterweise in den erfindungsgemäßen Zusammensetzungen enthalten sein. Gemäß einer besonderen Ausführungsform enthält die kosmetische Zusammensetzung weiterhin mindestens ein UV-Filter. Aufgrund des angenehmeren Hautgefühls bevorzugen viele Verbraucher Öl-in Wasser-Zusammensetzungen gegenüber Wasser-in-Öl-Emulsionen. Da die äußere Wasserphase in solchen Emulsionen häufig einen höheren Gewichtsanteil hat als die dispergierte Öl-/Fettphase, ist es vorteilhaft, die UV-Filtersubstanzen auf beide Phasen zu verteilen und also neben der mindestens einen öllöslichen auch mindestens eine wasserlösliche UV-Filtersubstanz einzuarbeiten. Besonders bevorzugt enthalten die erfindunsgemäßen Zusammensetzungen daher mindestens ein UV-Filter ausgewählt aus öllöslichen und wasserlöslichen organischen UV-Filtern. Gemäß einer anderen besonders bevorzugten Ausführungsform sind kosmetische Zusammensetzungen, enthaltend mindestens einen Reisextrakt und mindestens ein Salz von C₁₂₋₂₀-Alkylphosphat, insbesondere ein Salz von Cetylphosphat, und mindestens einen wasserlöslichen organischen UV-Filter. Diese Filter sind im weiteren genauer beschrieben.

Bevorzugte erfindungsgemäße kosmetische Zusammensetzungen sind **dadurch gekennzeichnet, dass** mindestens ein wasserlöslicher organischer UV-Filter in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, enthalten ist, jeweils bezogen auf das Gesamtgewicht der erfindungsgemäßen Zusammensetzung.

In einer weiteren bevorzugten Ausführungsform der Erfindung umfasst der mindestens eine wasserlösliche organische UV-Filter mindestens ein Derivat der Benzimidazolsulfonsäure.

Erfindungsgemäß bevorzugte Derivate der Benzimidazolsulfonsäure als wasserlösliche organische UV-Filtersubstanz(en) ist bzw. sind die Phenylen-1,4-bis-(2-benzimidazyl)-3,3'-5,5'-tetrasulfonsäure (UV-A) und ihre Salze, insbesondere die Alkali-, Erdalkali-, Ammonium-, Alkylammonium-, Alkanolammonium- und Glucammoniumsalze, bevorzugt die entsprechenden Natrium-, Kalium-, Trialkylammonium- oder Triethanolamin-Salze. Ebenfalls bevorzugt sind die Salze dieser Säure mit Amino-C₁-C₆-Alkanolen und Amino-C₁-C₆-Alkandiolen, insbesondere mit 2-Amino-2-methylpropan-1-ol, 2-Amino-2-methylpropan-1,3-diol, 2-Aminopropan-1-ol, 3-Aminopropan-1-ol, 1-Aminopropan-2-ol (MIPA) und 2-Amino-2-(hydroxymethyl)propan-1,3-diol (TRIS), wobei die Salze mit 2-Amino-2-methylpropan-1-ol und 2-Amino-2-methylpropan-1,3-diol besonders bevorzugt sind. Außerordentlich bevorzugt ist das Phenylen-1,4-bis-(2-benzimidazyl)-3,3'-5,5'-tetrasulfonsäure-bis-natriumsalz mit der INCI-Bezeichnung "Disodium Phenyl Dibenzimidazole Tetrasulfonate" (CAS-Nr.: 180898-37-7), das beispielsweise unter der Handelsbezeichnung Neo Heliopan AP von Symrise erhältlich ist. Weitere bevorzugte wasserlösliche organische UV-Filtersubstanzen sind die 2-Phenylbenzimidazol-5-sulfonsäure (INCI-Bezeichnung "Phenylbenzimidazole sulfonic acid" (CAS.-Nr. 27503-81-7), beispielsweise unter den Handelsnamen Neo Heliopan Hydro von Symrise oder Eusolex 232 von Merck KGaA erhältlich; UV-B-Filter) und ihre Salze, insbesondere die Alkali-, Erdalkali-, Ammonium-, Alkylammonium-, Alkanolammonium- und Glucammoniumsalze, bevorzugt die entsprechenden Natrium-, Kalium-, Trialkylammonium- oder Triethanolamin-Salze, weiterhin die Salze dieser Säure mit Amino-C₁-C₆-Alkanolen und Amino-C₁-C₆-Alkandiolen, insbesondere mit 2-Amino-2-methylpropan-1-ol, 2-Amino-2-methylpropan-1,3-diol, 2-Aminopropan-1-ol, 3-Aminopropan-1-ol und 1-Aminopropan-2-ol (MIPA), wobei die Salze mit 2-Amino-2-methylpropan-1-ol und 2-Amino-2-methylpropan-1,3-diol besonders bevorzugt sind, insbesondere aber die Salze der 2-Phenylbenzimidazol-5-sulfonsäure mit Natriumionen und mit 2-Amino-2-methylpropan-1-ol oder 2-Amino-2-methylpropan-1,3-diol. In einer besonders bevorzugten Ausführungsform der Erfindung ist der mindestens eine wasserlösliche organische UV-Filter, der ein Derivat der Benzimidazolsulfonsäure darstellt, ausgewählt aus Phenylen-1,4-bis-(2-benzimidazyl)-3,3'-5,5'-tetrasulfonsäure, 2-Phenylbenzimidazol-5-sulfonsäure und den Salzen dieser Säuren, bevorzugt den entsprechenden Natrium-, Kalium- oder Triethanolammonium-Salzen, insbesondere aus Phenylbenzimidazolsulfonsäure und dem Phenylen-1,4-bis-(2-benzimidazyl)-3,3'-5,5'-tetrasulfonsäure-bis-natriumsalz. Bevorzugte erfindungsgemäße Zusammensetzungen sind **dadurch gekennzeichnet, dass** das/die Derivat/e der Benzimidazolsulfonsäure in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, enthalten ist/sind, jeweils bezogen auf das Gesamtgewicht der erfindungsgemäßen Zusammensetzung.

Die Gewichtsangabe bezieht sich dabei, unabhängig von der Form, in der das Benzimidazolsulfonsäure-Derivat vorliegt (Salz, freie Säure), auf das Gewicht des Benzimidazolsulfonsäure-Derivats in der Säureform. Weitere bevorzugte erfindungsgemäße Zusammensetzungen sind dadurch gekennzeichnet, dass 2-Phenylbenzimidazol-5-sulfonsäure oder ein Salz hiervon in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, enthalten ist/sind, jeweils bezogen auf das Gesamtgewicht der erfindungsgemäßen Zusammensetzung. Weitere bevorzugte erfindungsgemäße Zusammensetzungen sind **dadurch gekennzeichnet, dass** Phenylen-1,4-bis-(2-benzimidazyl)-3,3'-5,5'-tetrasulfonsäure oder ein Salz hiervon in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, enthalten ist/sind, jeweils bezogen auf das Gesamtgewicht der erfindungsgemäßen Zusammensetzung, wobei die Gesamtmenge aller Benzimidazolsulfonsäure-Derivate 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, beträgt. Weitere bevorzugte erfindungsgemäße Zusammensetzungen sind **dadurch gekennzeichnet, dass** Phenylen-1,4-bis-(2-benzimidazyl)-3,3'-5,5'-tetrasulfonsäure und 2-Phenylbenzimidazol-5-sulfonsäure oder Salze hiervon in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, enthalten ist/sind, jeweils bezogen auf das Gesamtgewicht der erfindungsgemäßen Zusammensetzung, wobei die Gesamtmenge aller Benzimidazolsulfonsäure-Derivate 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, beträgt.

Gemäß einer anderen besonders bevorzugten Ausführungsform enthalten die erfindungsgemäßen kosmetischen Zusammensetzungen, neben mindestens einem Reisextrakt und mindestens einem Salz von C₁₂₋₂₀-Alkylphosphat, insbesondere ein Salz von Cetylphosphat, mindestens einen öllösliche organische UV-Filter, bevorzugt Polysilicone-15.

Die Substanz mit der INCI-Bezeichnung Polysilicone-15 wird auch als 3-(4-(2,2-Bis-Ethoxycarbonylvinyl)-phenoxy)propenyl)-methoxysiloxan/Dimethylsiloxan-Copolymer mit der Parsol^{®} SLX), Dimethicodiethylbenzalmalonat, Diethylbenzylidene Malonate Dimethicone oder Diethylmalonylbenzylidene Oxypropene Dimethicone bezeichnet und ist unter dem Handelsnamen Parsol^{®} SLX (INCI-Bezeichnung Dimethicodiethylbenzal malonate (CAS-Nr. 207574-74-1)) von DSM erhältlich. Die chemische Struktur ist beispielsweise in EP 709080 A2 beschrieben. Polysilicone-15 ist bevorzugt in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, enthalten, jeweils bezogen auf das Gesamtgewicht der erfindungsgemäßen Zusammensetzung. Gemäß einer bevorzugten Ausführungsform enthalten die kosmetischen Zusammensetzungen weiterhin mindestens ein C₁₄₋₂₀-Mono- oder Diacylglycerid, bevorzugt mindestens ein C₁₆₋₁₈-Mono- oder Diacylglycerid, besonders bevorzugt ausgewählt aus gehärteten Palmölglyceriden. Somit enthält die Zusammensetzung
a. mindestens einen Reisextrakt
b) mindestens einen UV-Filter, ausgewählt aus öllöslichen und wasserlöslichen organischen UV-Filtern,
c) mindestens einen anionischen Öl-in-Wasser-Emulgator, ausgewählt aus den Salzen von C₁₂₋₂₀-Alkylphosphat, insbesondere aus den Salzen von Cetylphosphat,
d) mindestens ein C₁₄₋₂₀-Mono- oder Diacylglycerid, bevorzugt mindestens ein C₁₆₋₁₈-Mono-oder Diacylglycerid, besonders bevorzugt ausgewählt aus gehärteten Palmölglyceriden,

Bevorzugt weisen die Zusammensetzungen einen pH-Wert bei 20°C von 6,0 - 8,0, auf. Erfindungsgemäß bevorzugte C₁₄₋₂₀-Mono- oder Diacylglyceride, die den zweiten Teil des erfindungsgemäßen O/W-Emulgatorsystems bilden, sind ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid und Diarachinoylglycerid. Weitere erfindungsgemäß bevorzugte C₁₄₋₂₀-Mono- oder Diacylglyceride sind Glyceride gehärteter, das heißt, hydrierter, bevorzugt vollständig hydrierter, Fettsäuren natürlicher Öle. Erfindungsgemäß besonders bevorzugt sind gehärtete Palmölglyceride.

Eine erfindungsgemäß besonders bevorzugtes O/W-Emulgatorsystem, umfassend ein Gemisch aus Dikaliummonocetylphosphat und Kaliumdicetylphosphat mit gehärteten Palmölglyceriden, ist als Handelsprodukt "Emulsiphos 677660" (INCI: Potassium Cetyl Phosphate, Hydrogenated Palm Glycerides) von der Firma Symrise erhältlich. Das O/W-Emulgatorsystem, bestehend aus mindestens einem Salz von C₁₂₋₂₀-Alkylphosphat als anionischem Öl-in-Wasser-Emulgator und mindestens einem C₁₄₋₂₀-Mono- oder Diacylglycerid, ist bevorzugt in einer Gesamtmenge von 0,5 Gew.-% bis 10 Gew.-%, besonders bevorzugt 1 bis 7 Gew.-%, insbesondere 2,5 - 4,5 Gew.-% und außerordentlich bevorzugt 3 - 4 Gew.-%, enthalten, jeweils bezogen auf das Gesamtgewicht der erfindungsgemäßen Zusammensetzung. Eine Stabilisierung des Lichtschutzfaktors (SPF) im vorstehend angegebenen pH-Bereich in Kombination mit dem mindestens einen anionischen Öl-in-Wasser-Emulgator, ausgewählt aus den Salzen von C₁₂₋₂₀-Alkylphosphat, und dem mindestens einen C₁₄₋₂₀-Mono-, Di- oder Triacylglycerid konnte insbesondere in Gegenwart des öllöslichen organischen UV-Filters Polysilicone-15 beobachtet werden.

Weitere bevorzugte erfindungsgemäße Zusammensetzungen sind **dadurch gekennzeichnet, dass** sie ein Gemisch aus Dikaliummonocetylphosphat und Kaliumdicetylphosphat mit gehärteten Palmölglyceriden und weiterhin Polysilicone-15 enthalten sowie einen pH-Wert bei 20°C von 6,0 - 8, bevorzugt 6,5 - 7,5, besonders bevorzugt 6,8 bis 7,3, aufweisen.

Weitere bevorzugte erfindungsgemäße Zusammensetzungen sind **dadurch gekennzeichnet, dass** sie ein Gemisch aus Dikaliummonocetylphosphat und Kaliumdicetylphosphat mit gehärteten Palmölglyceriden in einer Gesamtmenge von 0,5 Gew.-% bis 10 Gew.-%, besonders bevorzugt 1 bis 7 Gew.-%, insbesondere 2,5 - 4,5 Gew.-% und außerordentlich bevorzugt 3 - 4 Gew.-% und weiterhin Polysilicone-15 in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, jeweils bezogen auf das Gesamtgewicht der erfindungsgemäßen Zusammensetzung, enthalten sowie einen pH-Wert bei 20°C von 6,0 - 8, bevorzugt 6,5 - 7,5, besonders bevorzugt 6,8 bis 7,3, aufweisen. Weitere bevorzugte erfindungsgemäße Zusammensetzungen sind **dadurch gekennzeichnet, dass** sie ein Gemisch aus Dikaliummonocetylphosphat und Kaliumdicetylphosphat mit gehärteten Palmölglyceriden, weiterhin Polysilicone-15 und Natrium-2-Phenyl-benzimidazol-5-sulfonsäure enthalten sowie einen pH-Wert bei 20°C von 6,0 - 8, bevorzugt 6,5 - 7,5, besonders bevorzugt 6,8 bis 7,3, aufweisen. Weitere bevorzugte erfindungsgemäße Zusammensetzungen sind dadurch gekennzeichnet, dass sie ein Gemisch aus Dikaliummonocetylphosphat und Kaliumdicetylphosphat mit gehärteten Palmölglyceriden in einer Gesamtmenge von 0,5 Gew.-% bis 10 Gew.-%, besonders bevorzugt 1 bis 7 Gew.-%, insbesondere 2,5 - 4,5 Gew.-% und außerordentlich bevorzugt 3 - 4 Gew.-%, weiterhin Polysilicone-15 in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, und Natrium-2-Phenylbenzimidazol-5-sulfonsäure in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung, enthalten sowie einen pH-Wert bei 20°C von 6,0 - 8, bevorzugt 6,5 - 7,5, besonders bevorzugt 6,8 bis 7,3, aufweisen. Weitere bevorzugte erfindungsgemäße Zusammensetzungen sind dadurch gekennzeichnet, dass sie ein Gemisch aus Dikaliummonocetylphosphat und Kaliumdicetylphosphat mit gehärteten Palmölglyceriden, weiterhin Polysilicone-15 und Natrium-2-Phenyl-benzimidazol-5-sulfonsäure enthalten sowie einen pH-Wert bei 20°C von 6,0 - 8, bevorzugt 6,5 - 7,5, besonders bevorzugt 6,8 bis 7,3, aufweisen. Weitere bevorzugte erfindungsgemäße Zusammensetzungen sind dadurch gekennzeichnet, dass sie ein Gemisch aus Dikaliummonocetylphosphat und Kaliumdicetylphosphat mit gehärteten Palmölglyceriden in einer Gesamtmenge von 0,5 Gew.-% bis 10 Gew.-%, besonders bevorzugt 1 bis 7 Gew.-%, insbesondere 2,5 - 4,5 Gew.-% und außerordentlich bevorzugt 3 - 4 Gew.-%, weiterhin Polysilicone-15 in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, und Natrium-2-Phenylbenzimidazol-5-sulfonsäure in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung, enthalten sowie einen pH-Wert bei 20°C von 6,0 - 8, bevorzugt 6,5 - 7,5, besonders bevorzugt 6,8 bis 7,3, aufweisen. Weitere bevorzugte erfindungsgemäße Zusammensetzungen sind dadurch gekennzeichnet, dass sie ein Gemisch aus Dikaliummonocetylphosphat und Kaliumdicetylphosphat mit gehärteten Palmölglyceriden, weiterhin Polysilicone-15, das Natriumsalz und das 2-Amino-2-methylpropan-1-ol-Salz der 2-Phenylbenzimidazol-5-sulfonsäure enthalten sowie einen pH-Wert bei 20°C von 6,0 - 8, bevorzugt 6,5 - 7,5, besonders bevorzugt 6,8 bis 7,3, aufweisen.

Weitere bevorzugte erfindungsgemäße Zusammensetzungen sind dadurch gekennzeichnet, dass sie ein Gemisch aus Dikaliummonocetylphosphat und Kaliumdicetylphosphat mit gehärteten Palmölglyceriden in einer Gesamtmenge von 0,5 Gew.-% bis 10 Gew.-%, besonders bevorzugt 1 bis 7 Gew.-%, insbesondere 2,5 - 4,5 Gew.-% und außerordentlich bevorzugt 3 - 4 Gew.-%, weiterhin Polysilicone-15 in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, das Natriumsalz und das 2-Amino-2-methylpropan-1-ol-Salz der 2-Phenylbenzimidazol-5-sulfonsäure in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung, enthalten sowie einen pH-Wert bei 20°C von 6,0 - 8, bevorzugt 6,5 - 7,5, besonders bevorzugt 6,8 bis 7,3, aufweisen.

Gemäß den Herstellerangaben aus WO 2007/135196 A2 lassen sich die vorgenannten Derivate der Benzimidazolsulfonsäure gegenüber einer Kristallisation besonders gut durch Neutralisation mit basischen Aminosäuren, insbesondere mit Ornithin, Lysin, Arginin und/oder Histidin stabilisieren, wobei Arginin besonders bevorzugt ist. Dies konnte für den Gegenstand der vorliegenden Anmeldung nicht bestätigt werden. In einer bevorzugten Ausführungsform enthalten die erfindungsgemäßen bzw. die erfindungsgemäß verwendeten Zusammen-setzungen daher basische Aminosäuren, wie Lysin, Arginin und Histidin, in einer Gesamtmenge von 0 bis maximal 0,1 Gew.-%, bezogen auf die gesamte Zusammensetzung.

Eine Stabilisierung des Lichtschutzfaktors (SPF) im vorstehend angegebenen pH-Bereich in Kombination mit dem mindestens einen anionischen Öl-in-Wasser-Emulgator, ausgewählt aus den Salzen von C₁₂₋₂₀-Alkylphosphat, und dem mindestens einen C₁₄₋₂₀-Mono- oder Diacylglycerid konnte insbesondere beobachtet werden, wenn neben dem öllöslichen organischen UV-Filter Polysilicone-15 weiterhin mindestens ein Alkyl- und/oder Alkoxy-substituiertes Dibenzoylmethanderivat als öllöslicher organischer UV-Filter enthalten ist.

In einer bevorzugten Ausführungsform der Erfindung sind die erfindungsgemäßen bzw. die erfindungsgemäß verwendeten Zusammensetzungen, **dadurch gekennzeichnet, dass** der mindestens eine öllösliche UV-Filter eine Kombination aus mindestens einem Alkyl- und/oder Alkoxy-substituierten Dibenzoylmethanderivat und Polysilicone-15 umfasst.

Erfindungsgemäß bevorzugte Alkyl- und/oder Alkoxy-substituierte Dibenzoylmethanderivate sind **dadurch gekennzeichnet, dass** einer der Phenylreste mit mindestens einer Alkylgruppe, ausgewählt aus Methyl, Ethyl, n-Propyl, Isopropyl, n-Butyl, sec-Butyl, tert.-Butyl, n-Pentyl, Isopentyl, Neopentyl, 2-Ethylhexyl, und der andere Phenylrest mit mindestens einer Alkoxy-Gruppe, ausgewählt aus Methoxy, Ethoxy, n-Propoxy, Isopropoxy, n-Butoxy, sec-Butoxy, tert.Butoxy, n-Pentoxy, Isopentoxy, Neopentoxyl, 2-Ethylhexoxy, substituiert ist. Besonders bevorzugte Substituenten sind Isopropyl, tert.-Butyl und Methoxy. Erfindungsgemäß bevorzugte Alkyl- und/oder Alkoxy-substituierte Dibenzoylmethanderivate sind ausgewählt aus 2-Methyldibenzoylmethan, 4-Methyldibenzoylmethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyldibenzoylmethan, 4,4'-Dimethoxydibenzoylmethan, 4-tert-Butyl-4'-Methoxydibenzoylmethan, 2-Methyl-5-isopropyl-4'-methoxydibenzoyl-methan, 2-Methyl-5-tert-butyl-4'-methoxydibenzoylmethan, 2,4-Dimethyl-4'-methoxydibenzoyl-methan, 2,6-Dimethyl-4-tert-butyl-4'-methoxydibenzoylmethan. Besonders bevorzugt ist 4-tert-Butyl-4'-Methoxydibenzoylmethan mit der INCI-Bezeichnung Butyl Methoxydibenzoylmethane (auch als 1-(4'-tert-Butylphenyl)-3-(4'-methoxyphenyl)propan-1,3-dion bezeichnet), ein öllöslicher organischer Lichtschutzfilter, der z. B. als Parsol^{®} 1789 von DSM oder als Eusolex^{®} 9020 von Merck KGaA erhältlich ist. Bevorzugte erfindungsgemäße Zusammensetzungen sind **dadurch gekennzeichnet, dass** das mindestens eine Alkyl- und/oder Alkoxy-substituierte Dibenzoylmethanderivat in einer Gesamtmenge von 0,5 Gew.-% bis 20 Gew.-%, bevorzugt 1 bis 15 Gew.-%, besonders bevorzugt 2 bis 10 Gew.-% und außerordentlich bevorzugt 3 - 5 Gew.-%, enthalten ist, jeweils bezogen auf das Gesamtgewicht der erfindungsgemäßen Zusammensetzung. Weitere bevorzugte erfindungsgemäße Zusammensetzungen sind **dadurch gekennzeichnet, dass** das mindestens eine Alkyl- und/oder Alkoxy-substituiertes Dibenzoylmethanderivat ausgewählt ist aus 4-(tert.-Butyl)-4'-methoxydibenzoylmethan.

Weitere bevorzugte erfindungsgemäße Zusammensetzungen sind **dadurch gekennzeichnet, dass** 4-(tert.-Butyl)-4'-methoxydibenzoylmethan in einer Gesamtmenge von 0,5 Gew.-% bis 20 Gew.-%, bevorzugt 1 bis 15 Gew.-%, besonders bevorzugt 2 bis 10 Gew.-% und außerordentlich bevorzugt 3 - 5 Gew.-%, enthalten ist, jeweils bezogen auf das Gesamtgewicht der erfindungsgemäßen Zusammensetzung. Weitere bevorzugte erfindungsgemäße Zusammensetzungen sind **dadurch gekennzeichnet, dass** a) 4-(tert.-Butyl)-4'-methoxydibenzoylmethan, b) Polysilicone-15 und c) 2-Phenylbenzimidazol-5-sulfonsäure(salz) enthalten sind. Weitere bevorzugte erfindungsgemäße Zusammensetzungen sind **dadurch gekennzeichnet, dass** a) 4-tert-Butyl-4'-Methoxydibenzoylmethan, b) Polysilicone-15 und c) Dinatriumphenylbenzimidazoltetrasulfonat enthalten sind. Weitere bevorzugte erfindungsgemäße Zusammensetzungen sind **dadurch gekennzeichnet, dass** die UV-Filter a), b) und c) in einem Gewichtsverhältnis zueinander von a) : b) : c) wie (0,8 -1,5): (0,8 - 1,5) : (0,8 - 1,5) enthalten sind.

Weitere bevorzugte erfindungsgemäße Zusammensetzungen sind **dadurch gekennzeichnet, dass** die UV-Filter a), b) und c) in einem Gewichtsverhältnis zueinander von a) : b) : c) wie (0,9 - 1,2) : (0,9 - 1,2): (0,9 - 1,2), bevorzugt (1 - 1,1) : (1 - 1,1) : (1 - 1,1), enthalten sind.

Weitere bevorzugte erfindungsgemäße Zusammensetzungen sind **dadurch gekennzeichnet, dass** sie ein Gemisch aus Dikaliummonocetylphosphat und Kaliumdicetylphosphat mit gehärteten Palmölglyceriden, weiterhin Polysilicone-15, 4-tert-Butyl-4'-Methoxydibenzoylmethan und das Natriumsalz der 2-Phenylbenzimidazol-5-sulfonsäure enthalten sowie einen pH-Wert bei 20°C von 6,0 - 8, bevorzugt 6,5 - 7,5, besonders bevorzugt 6,8 bis 7,3, aufweisen.

Weitere bevorzugte erfindungsgemäße Zusammensetzungen sind **dadurch gekennzeichnet, dass** sie ein Gemisch aus Dikaliummonocetylphosphat und Kaliumdicetylphosphat mit gehärteten Palmölglyceriden in einer Gesamtmenge von 0,5 Gew.-% bis 10 Gew.-%, besonders bevorzugt 1 bis 7 Gew.-%, insbesondere 2,5 - 4,5 Gew.-% und außerordentlich bevorzugt 3 - 4 Gew.-%, weiterhin Polysilicone-15 in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, 4-tert-Butyl-4'-Methoxydibenzoylmethan in einer Gesamtmenge von 0,5 Gew.-% bis 20 Gew.-%, bevorzugt 1 bis 15 Gew.-%, besonders bevorzugt 2 bis 10 Gew.-% und außerordentlich bevorzugt 3 - 5 Gew.-% und das Natriumsalz der 2-Phenylbenzimidazol-5-sulfonsäure in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung, enthalten sowie einen pH-Wert bei 20°C von 6,0 - 8, bevorzugt 6,5 - 7,5, besonders bevorzugt 6,8 bis 7,3, aufweisen. Weitere bevorzugte erfindungsgemäße Zusammensetzungen sind **dadurch gekennzeichnet, dass** sie ein Gemisch aus Dikaliummonocetylphosphat und Kaliumdicetylphosphat mit gehärteten Palmölglyceriden, weiterhin Polysilicone-15, 4-tert-Butyl-4'-Methoxydibenzoylmethan, das Natriumsalz und das 2-Amino-2-methylpropan-1-ol-Salz der 2-Phenylbenzimidazol-5-sulfonsäure enthalten sowie einen pH-Wert bei 20°C von 6,0 - 8, bevorzugt 6,5 - 7,5, besonders bevorzugt 6,8 bis 7,3, aufweisen. Weitere bevorzugte erfindungsgemäße Zusammensetzungen sind **dadurch gekennzeichnet, dass** sie ein Gemisch aus Dikaliummonocetylphosphat und Kaliumdicetylphosphat mit gehärteten Palmölglyceriden in einer Gesamtmenge von 0,5 Gew.-% bis 10 Gew.-%, besonders bevorzugt 1 bis 7 Gew.-%, insbesondere 2,5 - 4,5 Gew.-% und außerordentlich bevorzugt 3 - 4 Gew.-%, weiterhin Polysilicone-15 in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, 4-tert-Butyl-4'-Methoxydibenzoyl-methan in einer Gesamtmenge von 0,5 Gew.-% bis 20 Gew.-%, bevorzugt 1 bis 15 Gew.-%, besonders bevorzugt 2 bis 10 Gew.-% und außerordentlich bevorzugt 3 - 5 Gew.-%, das Natriumsalz und das 2-Amino-2-methylpropan-1-ol-Salz der 2-Phenylbenzimidazol-5-sulfonsäure in einer Gesamtmenge von 0,5 Gew.-% bis 15 Gew.-%, bevorzugt 1 bis 10 Gew.-%, besonders bevorzugt 2 bis 5 Gew.-% und außerordentlich bevorzugt 3 - 4 Gew.-%, jeweils bezogen auf das Gesamtgewicht der erfindungsgemäßen Zusammensetzung, enthalten sowie einen pH-Wert bei 20°C von 6,0 - 8, bevorzugt 6,5 - 7,5, besonders bevorzugt 6,8 bis 7,3, aufweisen. Eine Stabilisierung des Lichtschutzfaktors (SPF) im vorstehend angegebenen pH-Bereich in Kombination mit dem mindestens einen anionischen Öl-in-Wasser-Emulgator, ausgewählt aus den Salzen von C₁₂₋₂₀-Alkylphosphat, und dem mindestens einen C₁₄₋₂₀-Mono-, Di-oder Triacylglycerid konnte insbesondere beobachtet werden, wenn neben dem öllöslichen organischen UV-Filter Polysilicone-15 weiterhin mindestens ein Alkyl- und/oder Alkoxy-substituiertes Dibenzoylmethanderivat als öllöslicher organischer UV-Filter enthalten ist. Um den Lichtschutzfaktor weiter zu erhöhen, können die erfindungsgemäßen bzw. die erfindungsgemäß verwendeten Zusammensetzungen bevorzugt mindestens eine weitere organische und/oder anorganische UV-Filtersubstanz enthalten.

Für solche erfindungsgemäße weitere UV-Filtersubstanzen haltige Mittel wird Schutz begehrt wird oder kann Schutz begehrt werden; weitere UV-Filtersubstanzen tragen zum technischen Ziel der Erfindung und somit zur Lösung der der anmeldungsgemäßen Erfindung zugrunde liegenden technischen Aufgabe bei. Bevorzugte weitere UV-Filtersubstanzen, die Mengen, in denen sie in erfindungsgemäßen Zusammensetzungen enthalten sind sind im Prioritätsdokument DE 10 2009 026414 auf den Seiten 74 bis 80 offenbart, die dort genannten Merkmale gehören eindeutig implizit zur Beschreibung der in der eingereichten Anmeldung enthaltenen Erfindung und damit zum Offenbarungsgehalt dieser Anmeldung.

Um die haptischen Eigenschaften und/oder den Lichtschutzfaktor der erfindungsgemäßen bzw. die erfindungsgemäß verwendeten Zusammensetzungen weiter zu verbessern, können die erfindungsgemäßen bzw. die erfindungsgemäß verwendeten Zusammensetzungen bevorzugt mindestens einen teilchenförmigen Feststoff, ausgewählt aus färbenden, farbgebenden, mattierenden oder glanzgebenden Pigmenten. Für erfindungsgemäße pigmenthaltige Mittel wird Schutz begehrt wird oder kann Schutz begehrt werden; Pigmente tragen zum technischen Ziel der Erfindung und somit zur Lösung der der anmeldungsgemäßen Erfindung zugrunde liegenden technischen Aufgabe bei. Bevorzugte Pigmente, die Mengen, in denen sie in erfindungsgemäßen Zusammensetzungen enthalten sind sind im Prioritätsdokument DE 10 2009 026414 auf den Seiten 81 bis 84 offenbart, die dort genannten Merkmale gehören eindeutig implizit zur Beschreibung der in der eingereichten Anmeldung enthaltenen Erfindung und damit zum Offenbarungsgehalt dieser Anmeldung.

Ein weiterer Gegenstand der vorliegenden Erfindung ist ein nicht-therapeutisches zur Verringerung der Porengröße der Haut, **dadurch gekennzeichnet, dass** ein Reisextrakt oder eine kosmetische oder dermatologische Zusammensetzung, enthaltend einen Reisextrakt, topisch auf die Haut aufgetragen wird. Bezüglich weiterer bevorzugter Ausführungsformen der erfindungsgemäßen Verfahren gilt mutatis mutandis das zu den erfindungsgemäß zu verwendenden Zusammensetzungen und Verwendungen Gesagte.

Die nachfolgenden Beispiele sollen den Gegenstand der Erfindung erläutern, ohne ihn hierauf zu beschränken. Alle Mengenangaben sind in Gew.-%, bezogen auf die gesamte Zusammensetzung.

### Wirknachweis:

Zwei Formulierungen (mit und ohne 1 Gew.-% Orsirtine GL) wurden an 10 Probanden im Bereich der Stirn für 6 Wochen zweimal täglich appliziert. Die Porengröße wurde vorher und nachher mittels konfokaler Laserscanning Mikroskopie gemessen und die durchschnittliche Porengröße durch Mittelung über alle Messwerte erhalten.

| | **1** | **2** |
|---|---|---|
| Cetiol CC | 3,000000 | 3,000000 |
| Montanov 68 | 5,000000 | 5,000000 |
| Caprylic/ Capric Triglyceride | 8,000000 | 8,000000 |
| Distelöl raffiniert | 2,000000 | 2,000000 |
| Cetearyl Alcohol | 0,500000 | 0,500000 |
| Cutina MD | 1,000000 | 1,000000 |
| Behenylalkohol | 1,000000 | 1,000000 |
| Controx KS | 0,050000 | 0,050000 |
| Vitamin E Acetat | 0,500000 | 0,500000 |
| Silikonöl 350 cs | 0,500000 | 0,500000 |
| Permulgin 3430 | 0,500000 | 0,500000 |
| Cetiol SB 45 | 1,000000 | 1,000000 |
| NOVATA AB PH | 1,000000 | 1,000000 |
| Generol R | 0,500000 | 0,500000 |
| Butylmethoxydibenzoylmethan | 1,000000 | 1,000000 |
| Uvinul T 150 | 1,250000 | 1,250000 |
| Propylparaben | 0,200000 | 0,200000 |
| Glycerin 86% | 5,000000 | 5,000000 |
| Hexandiol-1,6 | 6,000000 | 6,000000 |
| NTA-Na3 flüssig 40 % | 0,200000 | 0,200000 |
| Phenoxyethanol rein | 0,400000 | 0,400000 |
| Methylparaben | 0,200000 | 0,200000 |
| Tego Carbomer 140 | 0,400000 | 0,400000 |
| Orsirtine GL | 1,000000 | - |
| DSH- CN | 2,000000 | 2,000000 |
| Crossential SA 14 | 0,500000 | 0,500000 |
| Ederline LS | 2,000000 | 2,000000 |
| D-Panthenol 75 % | 2,000000 | 2,000000 |
| Parfum | 0,300000 | 0,300000 |
| Farbstoff | 0,000100 | 0,000100 |
| Aluminiumstärkeoctenylsuccinat | 1,000000 | 1,000000 |
| Relipidium | 0,500000 | 0,500000 |
| Simulgel NS | 0,500000 | 0,500000 |
| Natriumhydroxid | 0,030000 | 0,030000 |
| Wasser, vollentsalzt | ad 100,000000 | ad 100,000000 |

### Ergebnis:

| | Formulierung 1 (mit Orsirtine GL) | | | | Formulierung 2 (ohne Orsirtine GL) | | | |
|---|---|---|---|---|---|---|---|---|
| | Vorher | Nach | 6 | Wochen | Vorher | Nach | 6 | Wochen |
| | | Behandlungsdauer | | | | Behandlungsdauer | | |
| Porengröße (µm) | 383 | 329 | | | 359 | 353 | | |

| | | | | | | | | |
|---|---|---|---|---|---|---|---|---|
| Die Formulierung mit 1 Gew.-% Orsirtine GL zeigt eine hoch signifikante Reduktion der Porengröße um 14 % der Ausgangsporengröße. | | | | | | | | |

### 1. Oel-in-Wasser-Emulsionen

| 1. Beispielserie: | **1** | **2** | **3** | 3. Beispielserie: | **1** | **2** | **3** |
|---|---|---|---|---|---|---|---|
| Distelöl | 3,00 | 3,00 | 3,00 | Sisterna SP30-C | 2,0 | 2,0 | 2,0 |
| Myritol 318 | 5,00 | 5,00 | 5,00 | Sisterna SP70-C | 0,8 | 0,8 | 0,8 |
| Novata AB | 2,00 | 2,00 | 2,00 | Propylparaben | 0,2 | 0,2 | 0,2 |
| Lanette 22 | 1,00 | 1,00 | 1,00 | Methylparaben | 0,2 | 0,2 | 0,2 |
| Cutina MD | 2,00 | 2,00 | 2,00 | Ethanol | 5,0 | 3,0 | - |
| Stenol 1618 | 1,00 | 1,00 | 1,00 | Dry Flo Plus | 1,0 | 1,0 | 1,0 |
| Baysilon M350 | 1,00 | 1,00 | 1,00 | Parfum | 0,3 | 0,3 | 0,3 |
| Controx KS | 0,05 | 0,05 | 0,05 | Cetearyl Alcohol | 1,0 | 1,0 | 1,0 |
| Propylparaben | 0,20 | 0,20 | 0,20 | Fucogel 1000 | 2,0 | 1,0 | 0,5 |
| Dry Flo Plus | 1,00 | 2,00 | 3,00 | Keltrol SF | 0,2 | - | 0,2 |
| Propylenglycol | 5,00 | 5,00 | 5,00 | Aristoflex AVC | - | 0,5 | - |
| Glycerine | 5,00 | 3,00 | 3,00 | Hexandiol-1,6 | 5,0 | - | - |
| Methylparaben | 0,20 | 0,20 | 0,20 | Glycerin | 10,0 | 5,0 | 10,0 |
| Phenoxyethanol | 0,9 | 0,5 | 0,9 | Dow Corning 200 Fluid | 7,0 | - | - |
| Orsirtine GL | 0,1 | 0,5 | 1 | Dow Corning 245 | - | 5,0 | 5,0 |
| Parfum | 0,10 | 0,10 | 0,10 | Cetiol 868 | 2,0 | - | 2,0 |
| Aqua | ad. 100 | ad. 100 | ad. 100 | Orsirtine GL | 0,1 | 0,2 | 1 |
| | | | | Parfum | 0,3 | 0,3 | 0,3 |
| | | | | Wasser | Ad100 | Ad100 | Ad100 |

| 2. Beispielserie: | **1** | **2** | **3** | **4** |
|---|---|---|---|---|
| Lipoid S75-3 | 0,50 | 0,50 | 0,50 | 0,50 |
| Tocopherylacetat | 0,50 | 0,50 | 0,50 | 0,50 |
| Cutina MD | 1,00 | 1,00 | 1,00 | 1,00 |
| Lanette 22 | 2,00 | 2,00 | 2,00 | 2,00 |
| Baysilone M 350 | 0,50 | 0,50 | 0,50 | 0,50 |
| Propylparaben | 0,20 | 0,20 | 0,20 | 0,20 |
| Dow Corning 9040 | 1,00 | 1,00 | 1,00 | 1,00 |
| Glycerin | 4,50 | 3,00 | 3,00 | 3,00 |
| Hexandiol | 6,00 | 3,00 | - | 3,00 |
| Methylparaben | 0,20 | 0,20 | 0,20 | 0,20 |
| Tego Carbomer 140 | 0,30 | 0,30 | 0,30 | 0,30 |
| DS-HCN | 5,00 | 5,00 | 5,00 | 5,00 |
| Propylenglycol | 5,00 | 5,00 | 5,00 | 5,00 |
| Simulgel NS | 1,50 | 1,50 | 1,50 | 1,50 |
| TiO2 | 0,50 | 0,50 | 0,50 | 0,50 |
| Orsirtine GL | 0,001 | 0,1 | 1 | 0,01 |
| Wasser | Ad100 | Ad100 | Ad100 | Ad100 |

| 5. Beispielserie: | 1 | 2 | 3 | 4. Beispielserie: | **1** | **2** | **3** |
|---|---|---|---|---|---|---|---|
| Montanov 202 | 5,0 | 5,0 | 5,0 | Dow Corning 245 | 25 | 25 | 25 |
| | | | | Fluid | | | |
| Cutina MD | 2,0 | 2,0 | 2,0 | Abil EM 90 | 2 | 2 | 2 |
| Cetearyl Alcohol | 1,0 | 1,0 | 1,0 | Belsil DM 100 | 2,5 | 2,5 | 2,5 |
| Cetiol B | 9,0 | 9,0 | 9,0 | Hydrogenated | 2 | 2,5 | 2 |
| | | | | Castor Oil | | | |
| Controx KS | 0,05 | 0,05 | 0,05 | Glycerin | 5 | 5 | 5 |
| Dow Corning 245 | - | 7,0 | 3,0 | NaCl | 2 | 2,1 | 2 |
| Dow Corning 9040 | 1,0 | 1,0 | - | Symdiol 68 | 0,5 | 0,5 | 0,5 |
| Parfum | 0,3 | 0,3 | 0,3 | Phenoxyethanol | 0,4 | 0,4 | 0,4 |
| Fucogel 1000 | - | 2,0 | - | Orsirtine GL | 0,001 | 0,01 | 0,1 |
| Keltrol SF | - | 0,2 | 0,2 | Parfum | 0,3 | 0,3 | 0,3 |
| Aristoflex AVC | - | - | 0,5 | Wasser | Ad 100 | Ad 100 | Ad 100 |
| Hexandiol-1,6 | 6,0 | 6,0 | - | | | | |
| Glycerin | 3,0 | 5,0 | 10,0 | | | | |
| Dow Corning 200 Fluid | 7,0 | 7,0 | - | | | | |
| Dow Corning 245 | - | 5,0 | 3,0 | | | | |
| Dow Corning 9040 | 1,0 | - | 1,0 | | | | |
| Propylenglycol | 2,0 | 2,0 | - | | | | |
| Wasser | Ad100 | Ad100 | Ad100 | | | | |
| Tego Carbomer | 0,3 | 0,3 | 0,3 | | | | |
| Dry Flo Plus | 1,0 | 1,0 | 1,0 | | | | |
| Orsirtine GL | 1 | 0,1 | 2 | | | | |

| 6. Beispielserie: | **1** | **2** | **3** |
|---|---|---|---|
| Glucamate Sse-20 | 2,0 | 2,0 | 2,0 |
| Sympatens-BS/080 | 2,0 | 2,0 | 2,0 |
| Dry Flo Plus | - | 1,0 | 1,0 |
| Parfum | 0,3 | 0,3 | 0,3 |
| Silikonöl 350 | 0,3 | 0,3 | - |
| Cutina MD | 2,0 | 2,0 | 2,0 |
| Paraffinöl | 2,0 | 1,0 | - |
| Cetearyl Alcohol | 1,0 | 1,5 | 1,25 |
| Capryl-/Caprinsäure-Triglycerid | - | 3,5 | 3,0 |
| Keltrol SF | - | - | 0,2 |
| Aristoflex AVC | - | 0,5 | 0,5 |
| Hexandiol-1,6 | - | 5,0 | 5,0 |
| Glycerin | 2,0 | 5,0 | 10,0 |
| Culminal MHPC 100 | 0,1 | 0,1 | 0,1 |
| Propylenglycol | 2,0 | 5,0 | 3,0 |
| Orsirtine GL | 0,1 | 2 | 1 |
| Wasser | Ad100 | Ad100 | Ad100 |

| 7. schützende | 1 | 2 | 3 | 4 | 1 | 2 | 3 | 4 |
|---|---|---|---|---|---|---|---|---|
| Tagescremes | | | | | | | | |
| Distelöl | 3,00 | 3,00 | 3,00 | 3,00 | 3,00 | 3,00 | 3,00 | 3,00 |
| Myritol 318 | 5,00 | 5,00 | 5,00 | 5,00 | 5,00 | 5,00 | 5,00 | 5,00 |
| Novata AB | 2,00 | 2,00 | 2,00 | 2,00 | 2,00 | 2,00 | 2,00 | 2,00 |
| Behenyl Alcohol | 1,00 | 1,00 | 1,00 | 1,00 | 1,00 | 1,00 | 1,00 | 1,00 |
| Cutina MD | 2,00 | 2,00 | 2,00 | 2,00 | 2,00 | 2,00 | 2,00 | 2,00 |
| Cetearyl Alcohol | 1,00 | 1,00 | 1,00 | 1,00 | 1,00 | 1,00 | 1,00 | 1,00 |
| Isopropylstearat | 4,00 | 4,00 | 4,00 | 4,00 | 4,00 | 4,00 | 4,00 | 4,00 |
| Shea Butter | 2,00 | 2,00 | 2,00 | 2,00 | 2,00 | 2,00 | 2,00 | 2,00 |
| Baysilone-Öl M 350 | 1,00 | 1,00 | 1,00 | 1,00 | 1,00 | 1,00 | 1,00 | 1,00 |
| Controx KS | 0,05 | 0,05 | 0,05 | 0,05 | 0,05 | 0,05 | 0,05 | 0,05 |
| Propylparaben | 0,20 | 0,20 | 0,20 | 0,20 | 0,20 | 0,20 | 0,20 | 0,20 |
| Dow Corning 1501 Fluid | 1,00 | 1,00 | 1,00 | 1,00 | 1,00 | 1,00 | 1,00 | 1,00 |
| Dry Flo Plus | 1,00 | 1,00 | 1,00 | 1,00 | 1,00 | 1,00 | 1,00 | 1,00 |
| TiO₂ | 0,50 | 0,50 | 0,50 | 0,50 | 0,50 | 0,50 | 0,50 | 0,50 |
| Hexandiol | 6,00 | 3,00 | - | - | 6,00 | 3,00 | - | - |
| Propylenglycol | 5,00 | 5,00 | 5,00 | 5,00 | 5,00 | 5,00 | 5,00 | 5,00 |
| Glycerol | 5,00 | 3,00 | 3,00 | 3,00 | 5,00 | 3,00 | 3,00 | 3,00 |
| Methylparaben | 0,20 | 0,20 | 0,20 | 0,20 | 0,20 | 0,20 | 0,20 | 0,20 |
| Tego Carbomer | 0,40 | 0,40 | 0,40 | 0,40 | 0,40 | 0,40 | 0,40 | 0,40 |
| Orsirtine GL | 1 | 0,1 | 2 | 0,5 | 1 | 0,1 | 2 | 0,5 |
| Hydrovance | 4,30 | 2,50 | 8,60 | 10,00 | 4,30 | 2,50 | 8,60 | 10,00 |
| Ederline H | 1,0 | 2,0 | 1,0 | 0,5 | - | - | - | - |
| Ederline L | - | - | - | - | 1,0 | 2,0 | 1,0 | 0,5 |
| Phytokine | 2,00 | 1,50 | 2,00 | 2,00 | 2,00 | 1,50 | 2,00 | 2,00 |
| Ridulisse C | 0,50 | 0,50 | 0,50 | 0,50 | 0,50 | 0,50 | 0,50 | 0,50 |
| Liftiline | 2,00 | 3,00 | 2,00 | 3,00 | 2,00 | 3,00 | 2,00 | 3,00 |
| Matrixyl 3000 | - | 2,00 | - | - | - | 2,00 | - | - |
| Perfume | 0,10 | 0,10 | 0,10 | 0,10 | 0,10 | 0,10 | 0,10 | 0,10 |
| Aqua | ad 100 | ad 100 | ad 100 | ad 100 | ad 100 | ad 100 | ad 100 | ad 100 |

### 8. Hautcremes:

Die Zusammensetzungen 1 - 3 sind weitere bevorzugte Ausführungsformen von schützenden Tagescremes für besonders anspruchsvolle Haut mit einem Gehalt an Antiageing-Wirkstoffen.

| | 1 | 2 | 3 |
|---|---|---|---|
| Cetearyl Isononanoate | 4,00 | 4,00 | 4,00 |
| Mineral oil | 6,00 | 6,00 | 6,00 |
| Cutina CBS | 2,00 | 2,00 | 2,00 |
| Palmitic Acid / Stearic Acid | 1,50 | 1,50 | 1,50 |
| Eumulgin B3 | 1,00 | 1,00 | 1,00 |
| Baysilone-Öl M 350 | 1,00 | 1,00 | 1,00 |
| Tocopheryl Acetate | 0,50 | 0,50 | 0,50 |
| Propylparaben | 0,30 | 0,30 | 0,30 |
| Pemulen TR 1 | 0,27 | 0,27 | 0,27 |
| Glycerin | 5,00 | 3,00 | 3,00 |
| Milchsäure 80 % | 0,26 | 0,26 | 0,26 |
| Propylenglycol | 5,00 | 5,00 | 5,00 |
| Methylparaben | 0,30 | 0,30 | 0,30 |
| Phenoxyethanol | 0,90 | 0,90 | 0,90 |
| Phytokine | 2,00 | 1,50 | 2,00 |
| Ridulisse C | 0,50 | 0,50 | 0,50 |
| Liftiline | 2,00 | 3,00 | 2,00 |
| Sepigel 305 | 0,50 | 0,50 | 0,50 |
| Orsirtine GL | 0,1 | 0,2 | 0,5 |
| Taurin | 1,00 | 0,50 | 2,00 |
| Calmosensine | 1,00 | 2,00 | - |
| Keltrol SF | 0,20 | 0,20 | 0,20 |
| N,N-Bis-(2-hydroxyethyl)harnstoff | 4,30 | 2,50 | 8,60 |
| Perfume | 0,30 | 0,30 | 0,30 |
| Natrium Ascorbylphosphat | 1,00 | - | 1,00 |
| Keratec Pep | - | 2,00 | - |
| Kreatin | - | - | 1,00 |
| Retinol | 0,10 | - | - |
| Deliner | - | 2,00 | - |
| Wasser | ad 100 | ad 100 | ad 100 |

### 9. Tagescremes:

Die Zusammensetzungen 1 - 5 sind bevorzugte Ausführungsformen von schützenden Tagescremes für besonders anspruchsvolle Haut mit einem Gehalt an Antiageing-Wirkstoffen.

| | 1 | 2 | 3 | 4 | 5 |
|---|---|---|---|---|---|
| Lipoid S 75-3 | 0,50 | 0,50 | 0,50 | 0,50 | 0,50 |
| Isopropylstearate | 4,00 | 4,00 | 4,00 | 4,00 | 4,00 |
| Dibutyl Adipate | 2,00 | 2,00 | 2,00 | 2,00 | 2,00 |
| Tocopheryl Acetate | 0,50 | 0,50 | 0,50 | 0,50 | 0,50 |
| Cutina MD | 1,00 | 1,00 | 1,00 | 1,00 | 1,00 |
| Behenyl Alcohol | 2,00 | 2,00 | 2,00 | 2,00 | 2,00 |
| Baysilone-Öl M 350 | 0,50 | 0,50 | 0,50 | 0,50 | 0,50 |
| Propylparaben | 0,20 | 0,20 | 0,20 | 0,20 | 0,20 |
| Dow Corning 9040 | 1,00 | 1,00 | 1,00 | 1,00 | 1,00 |
| Glycerol | 3,00 | 4,50 | 3,00 | 3,00 | 3,00 |
| Hexandiol | 6,00 | 3,00 | - | - | 4,00 |
| Methylparaben | 0,20 | 0,20 | 0,20 | 0,20 | 0,20 |
| Tego Carbomer | 0,30 | 0,30 | 0,30 | 0,30 | 0,30 |
| Algae Extract | 1,00 | - | - | - | - |
| Photosomes | 0,20 | 0,20 | 0,20 | 0,20 | 0,20 |
| Dimethylmethoxy Chromanol | 0,01 | 0,01 | 0,01 | 0,01 | 0,01 |
| Propylenglycol | 5,00 | 5,00 | 5,00 | 5,00 | 5,00 |
| Phytokine | 2,00 | 1,50 | 2,00 | 2,00 | 1,50 |
| Ridulisse C | 0,50 | 0,50 | 0,50 | 0,50 | 0,50 |
| Liftiline | 2,00 | 3,00 | 2,00 | 2,00 | 3,00 |
| Orsirtine GL | 1,00 | 0,5 | 0,1 | 0,6 | 0,1 |
| Natrium Ascorbylphosphat | - | - | 1,00 | 1,00 | 1,00 |
| Natrulon RC50DG | - | - | - | - | 1,00 |
| Deliner | - | - | 2,00 | 1,00 | - |
| Matrixyl 3000 | 1,00 | 1,00 | - | - | 1,00 |
| Simulgel NS | 1,50 | 1,50 | 1,50 | 1,50 | 1,50 |
| Sodium Benzoate | - | 0,20 | 0,50 | - | - |
| Phenoxyethanol | 0,50 | - | - | | |
| Symdiol 68 | 0,30 | 0,50 | 0,30 | - | - |
| TiO₂ | 0,50 | 0,50 | - | - | - |
| Hydrovance | 2,50 | 4,30 | 8,60 | 8,60 | 8,60 |
| Silymarin Phytosome | 0,30 | - | - | - | 0,50 |
| Ectoin | 0,50 | - | 0,50 | 0,50 | 0,50 |
| Vitamin B6 | - | - | 0,01 | 0,01 | 0,01 |
| Perfume | 0,35 | 0,35 | 0,35 | 0,35 | 0,35 |
| Aqua | ad 100 | ad 100 | ad 100 | ad 100 | ad 100 |

### 10. Pflegecremes

Die Zusammensetzungen 1 - 6 sind bevorzugte Ausführungsformen von schützenden Pflegecremes mit gel-ähnlicher Konsistenz.

| | 1 | 2 | 3 | 4 | 5 | 6 |
|---|---|---|---|---|---|---|
| 2-Ethylhexylpalmitat | 2,00 | 2,00 | 2,00 | 2,00 | 2,00 | 2,00 |
| Dow Corning 1403 Fluid | 5,00 | 5,00 | 5,00 | 5,00 | 5,00 | 5,00 |
| Synperonic PE L 64 | 2,00 | 2,00 | 2,00 | 2,00 | 2,00 | 2,00 |
| Dow Corning 9040 | 5,00 | 5,00 | 5,00 | 5,00 | 5,00 | 5,00 |
| Dipropylenglykol | 5,00 | 5,00 | 5,00 | 5,00 | 5,00 | 5,00 |
| Tego Carbomer 140 | 0,70 | 0,70 | 0,70 | 0,70 | 0,70 | 0,70 |
| Parfum | 0,35 | 0,30 | 0,35 | 0,35 | 0,30 | 0,30 |
| Phenoxyethanol, rein | 0,40 | 0,40 | 0,40 | 0,40 | 0,40 | 0,40 |
| Natrulon RC-50DG | - | 0,75 | 0,75 | 0,75 | 0,75 | 0,75 |
| Menthyl Lactate | 0,50 | 0,50 | 0,50 | 0,50 | 0,50 | 0,50 |
| Ethanol 96 % DEP vergällt | 5,00 | 5,00 | 5,00 | 5,00 | 5,00 | 5,00 |
| Coffein wasserfrei | 0,50 | - | 0,50 | 0,50 | 0,50 | 0,50 |
| Ridulisse C | - | - | - | 0,50 | - | - |
| Orsirtine GL | 0,1 | 0,01 | 0,5 | 1 | 0,4 | 0,8 |
| Simulgel NS | 0,75 | 0,75 | 0,75 | 0,75 | 0,75 | 0,75 |
| Matrixyl | - | - | - | - | 2,00 | - |
| Matrixyl 3000 | - | - | - | - | - | 2,00 |
| Natriumhydroxid Perlen | 0,081 | 0,081 | 0,081 | 0,081 | 0,081 | 0,081 |
| Wasser, vollentsalzt | ad 100,00 | ad 100,00 | ad 100,00 | ad 100,00 | ad 100,00 | ad 100,00 |

### 11. Die Zusammensetzungen 1 - 5 sind bevorzugte Ausführungsformen von schützenden Tagescremes mit höherem Lichtschutzfilter (SPF ca. 10 - 15) für besonders anspruchsvolle Haut mit einem Gehalt an Antiageing-Wirkstoffen.

| | 1 | 2 | 3 | 4 | 5 |
|---|---|---|---|---|---|
| Montanov 68 | 5,00 | 5,00 | 5,00 | 5,00 | 5,00 |
| Myritol 318 | 5,00 | 5,00 | 5,00 | 5,00 | 5,00 |
| Cetiol SB 45 | 0,50 | 0,50 | 0,50 | 0,50 | 0,50 |
| Novata AB | 2,00 | 2,00 | 2,00 | 2,00 | 2,00 |
| Stenol 1618 | 1,00 | 1,00 | 1,00 | 1,00 | 1,00 |
| Baysilon M 350 | 0,50 | 0,50 | 0,50 | 0,50 | 0,50 |
| Tocopherylacetat | 0,50 | 0,50 | 0,50 | 0,50 | 0,50 |
| Controx KS | 0,25 | 0,25 | 0,25 | 0,25 | 0,25 |
| Parsol SLX | - | 4,00 | 4,00 | - | - |
| Parsol HS | - | 2,00 | 2,00 | 2,00 | - |
| Neo Heliopan AP | - | - | - | 1,00 | - |
| Parsol 340 | - | - | - | 5,00 | - |
| Uvinul MBC 95 | 2,00 | - | - | - | - |
| Parsol 1789 | 1,00 | 1,80 | 1,80 | - | - |
| Propylparaben | 0,20 | 0,20 | 0,20 | 0,20 | 0,20 |
| Tego Carbomer 140 | 0,50 | 0,50 | 0,50 | 0,50 | 0,50 |
| 1,6-Hexandiol | 6,00 | 6,00 | 3,00 | 6,00 | - |
| Talkum | 0,50 | 0,50 | 0,50 | 0,50 | 0,50 |
| Methylparaben | 0,20 | 0,20 | 0,20 | 0,20 | 0,20 |
| Glycerin | 4,50 | 4,50 | 3,00 | 4,50 | 3,00 |
| Dry Flo Plus | 1,00 | 1,00 | 1,00 | 1,00 | 1,00 |
| Natipide 2 PG | 1,00 | 1,00 | 1,00 | 1,00 | 1,00 |
| Phenoxyethanol | 0,40 | 0,40 | 0,40 | 0,40 | 0,40 |
| Orsirtine GL | 0,1 | 1 | 0,5 | 2,0 | 0,01 |
| Carnitin | - | 0,10 | 0,50 | - | - |
| Matrixyl 3000 | 1,00 | - | - | 4,00 | 2,00 |
| Parfum | 0,40 | 0,40 | 0,40 | 0,40 | 0,40 |
| Aqua | ad 100 | ad 100 | ad 100 | ad 100 | ad 100 |

Die Zusammensetzungen 12. 1 - 3 sind bevorzugte Ausführungsformen von schützenden Cremes auf der Basis einer reichhaltigen Wasser-in-Öl-Emulsion, die in die Haut einmassiert werden kann und dadurch einen zusätzlichen Bonuseffekt hervorruft. Die Zusammensetzungen 13.1 - 3 sind bevorzugte Ausführungsformen von schützenden Gesichtswässern, die bevorzugt mit einem Pad, Bausch oder Tuch auf die Haut appliziert werden.

| 12. W/O Emulsionen | 1 | 2 | 3 | 13. Gesichtswässer | 1 | 2 | 3 |
|---|---|---|---|---|---|---|---|
| Lameform TGI | 3,00 | 3,00 | 3,00 | Dipropylenglycol | 10,00 | 10,00 | 10,00 |
| Elfacos ST 37 | 0,50 | 0,50 | 0,50 | Chlorhexidindigluconat | 1,00 | 1,00 | 1,00 |
| Microcrystalline Wax | 3,00 | 3,00 | 3,00 | Synperonic PE7 L 64 | 3,00 | 3,00 | 3,00 |
| Softisan 649 | 1,00 | 1,00 | 1,00 | D-Panthenol | 0,50 | 0,50 | 0,50 |
| Paraffinöl | 8,00 | 8,00 | 8,00 | Hydagen CMF | 3,00 | 3,00 | 3,00 |
| Vaseline | 2,00 | 2,00 | 2,00 | PEG-40 Hydrogenated Castor Oil / Trideceth 9 / Propylene Glycol | 0,50 | 0,50 | 0,50 |
| Vitamin E Acetat | 2,00 | 2,00 | 2,00 | Caomint | 0,50 | 0,50 | 0,50 |
| Methylparaben | 0,30 | 0,30 | 0,30 | Orsirtine GL | 1 | 0,5 | 0,1 |
| Propylparaben | 0,30 | 0,30 | 0,30 | Ridulisse C | 1,00 | - | - |
| Isopropylisostearat | 8,00 | 8,00 | 8,00 | Fucogel 1000 | - | - | 1,00 |
| 1,3-Butylenglycol | 5,00 | 6,00 | 7,00 | Betain | - | 1,00 | - |
| Orsirtine GL | 0,50 | 1,0 | 5 | Parfum | 0,20 | 0,20 | 0,20 |
| Carnitin | - | 0,10 | 0,50 | Aqua | ad 100 | ad 100 | ad 100 |
| Milchsäure 80%ig | 0,60 | 0,60 | 0,60 | | | | |
| Panthenol | 0,5 | 1,0 | 0,5 | | | | |
| Tephrosia pupurea | 0,1 | - | - | | | | |
| Seed Extract | | | | | | | |
| Hydrovance | 2,00 | 4,00 | - | | | | |
| Parfum | 0,2 | 0,2 | 0,2 | | | | |
| Water | ad | ad | ad | | | | |
| | 100 | 100 | 100 | | | | |

### 14. Die Zusammensetzungen 1 - 3 sind bevorzugte Ausführungsformen von schützenden Cremes auf der Basis einer reichhaltigen und dennoch nicht-fettenden Wasser-in-Silicon-Emulsion, die in die Haut einmassiert werden kann.

| | 1 | 2 | 3 |
|---|---|---|---|
| Belsil DM 100 | 2,50 | 2,50 | 2,50 |
| Dow Corning 245 Fluid | 25,00 | 25,00 | 25,00 |
| Abil EM 90 | 2,00 | 2,00 | 2,00 |
| Natriumchlorid | 2,00 | 2,00 | 2,00 |
| Symdiol 68 | 0,30 | 0,30 | 0,30 |
| Phenoxyethanol | 0,40 | 0,40 | 0,40 |
| Orsirtine GL | 0,01 | 0,01 | 0,01 |
| Ridulisse C | - | 2,00 | 1,00 |
| Rhamnosoft | 2,00 | - | - |
| Hydrovance | - | 3,00 | - |
| Folsäure | - | - | 0,10 |
| Parfum | 0,30 | 0,30 | 0,30 |
| Aqua | ad 100 | ad 100 | ad 100 |

### 15. Beispielserie:

Die Zusammensetzungen 1 - 4 sind bevorzugte Ausführungsformen von schützenden Tagescremes für besonders anspruchsvolle Haut mit einem erhöhten Gehalt an diversen Antiageing-Wirkstoffen.

| | 1 | 2 | 3 | 4 |
|---|---|---|---|---|
| Distelöl | 3,00 | 3,00 | 3,00 | 3,00 |
| Myritol 318 | 5,00 | 5,00 | 5,00 | 5,00 |
| Novata AB | 2,00 | 2,00 | 2,00 | 2,00 |
| Behenyl Alcohol | 1,00 | 1,00 | 1,00 | 1,00 |
| Cutina MD | 2,00 | 2,00 | 2,00 | 2,00 |
| Cetearyl Alcohol | 1,00 | 1,00 | 1,00 | 1,00 |
| Isopropylstearat | 4,00 | 4,00 | 4,00 | 4,00 |
| Shea Butter | 2,00 | 2,00 | 2,00 | 2,00 |
| Baysilone-Öl M 350 | 1,00 | 1,00 | 1,00 | 1,00 |
| Controx KS | 0,05 | 0,05 | 0,05 | 0,05 |
| Propylparaben | 0,20 | 0,20 | 0,20 | 0,20 |
| Dow Corning 1501 Fluid | 1,00 | 1,00 | 1,00 | 1,00 |
| Dry Flo Plus | 1,00 | 1,00 | 1,00 | 1,00 |
| TiO₂ | 0,50 | 0,50 | 0,50 | 0,50 |
| Hexandiol | 6,00 | 3,00 | - | - |
| Propylenglycol | 5,00 | 5,00 | 5,00 | 5,00 |
| Glycerol | 5,00 | 3,00 | 3,00 | 3,00 |
| Methylparaben | 0,20 | 0,20 | 0,20 | 0,20 |
| Tego Carbomer | 0,40 | 0,40 | 0,40 | 0,40 |
| Orsirtine GL | 1,00 | 0,5 | 2,0 | 0,1 |
| Algenextrakt | 1,00 | - | - | - |
| Symdiol 68 | 0,30 | 0,50 | - | - |
| DSH-CN | 5,00 | 5,00 | 5,00 | 5,00 |
| Hydrovance | 4,30 | 2,50 | 8,60 | 10,00 |
| Matrixyl 3000 | - | 2,00 | - | - |
| Ridulisse C | - | - | 2,00 | 2,50 |
| Argireline | 1,00 | - | - | - |
| Sepilift DPHP | - | 0,10 | - | - |
| Ederline H | 2,00 | - | - | - |
| Perfume | 0,10 | 0,10 | 0,10 | 0,10 |
| Aqua | ad 100 | ad 100 | ad 100 | ad 100 |

| 16. Tagescreme: | | 17. Nachtcreme: | |
|---|---|---|---|
| Lipoid S 75-3 | 1,000000 | Montanov 68 | 3,000000 |
| Isopropylstearat | 2,000000 | Capryl-/Caprinsäure-Triglycerid | 2,000000 |
| Cetiol B | 4,000000 | Cetiol SN | 8,000000 |
| Vitamin E Acetat | 0,500000 | Safloröl raffiniert | 3,500000 |
| Cutina MD | 1,000000 | Cetearyl Alcohol | 2,500000 |
| Cetearyl Alcohol | 2,500000 | Cutina MD | 1,700000 |
| NOVATA AB PH | 0,500000 | Behenylalkohol | 3,000000 |
| Behenylalkohol | 3,500000 | Cetiol SB 45 | 4,000000 |
| Silikonöl 350 cs | 1,500000 | NOVATA AB PH | 0,500000 |
| Propylparaben | 0,200000 | Silikonöl 350 cs | 2,500000 |
| Dow Corning 9040 | 2,000000 | Controx KS | 0,050000 |
| Glycerin 86% | 8,000000 | Propylparaben | 0,200000 |
| Hexandiol-1,6 | 6,000000 | Glycerin 86% | 9,000000 |
| Methylparaben | 0,200000 | Hexandiol-1,6 | 6,000000 |
| Talkum | 3,000000 | Methylparaben | 0,200000 |
| Tego Carbomer 140 | 0,400000 | Tego Carbomer 140 | 0,300000 |
| Hydroglycolic Extract of | | | 1,000000 |
| Caviar | 1,000000 | Orsirtine GL | |
| Orsirtine GL | 0,500000 | Phenoxyethanol, rein | 0,400000 |
| Ederline L | 1,000000 | Ultrasomes | 0,100000 |
| DSH-C N | 3,000000 | Matrixyl 3000 | 3,000000 |
| Photosomes | 0,200000 | Parfum | 0,100000 |
| Lipochroman-6 | 0,010000 | FD&C Yellow No. 6 | 0,000700 |
| Propandiol-1,2 | 4,000000 | Aluminiumstärkeoctenylsuccinat | 1,800000 |
| Matrixyl 3000 | 3,000000 | Simulgel EPG | 0,400000 |
| Phenoxyethanol, rein | 0,400000 | Natriumhydroxid | 0,031000 |
| Parfum | 0,200000 | Wasser, vollentsalzt | ad 100 |
| RONASHERELDP | 1,000000 | | |
| FD&C Yellow No. 6 | 0,000700 | | |
| Simulgel NS | 1,500000 | | |
| Natriumhydroxid | 0,040000 | | |
| Wasser, vollentsalzt | ad 100,00000 | | |

### 18. Hautcremes auf Basis einer Lipoprotein-Creme

Die Zusammensetzungen 1 - 6 sind bevorzugte Ausführungsformen von schützenden Cremes mit besonderer Hautverträglichkeit und einem Gehalt an diversen Antiageing-Wirkstoffen, auf Basis einer besonders milden Emulsionsbasis mit einem natürlichen Protein-Emulgator (Hibiscin^{®} HP LS 9198).

| | 1 | 2 | 3 | 4 | 5 | 6 |
|---|---|---|---|---|---|---|
| Myritol^{®} PC | 3,5 | 3,5 | 3,5 | 3,5 | 3,5 | 3,5 |
| Lanette^{®} 22 | 3,0 | 3,0 | 3,0 | 3,0 | 3,0 | 3,0 |
| Cutina^{®} GMS-V | 3,0 | 3,0 | 3,0 | 3,0 | 3,0 | 3,0 |
| Stenol^{®} 16/18 | 2,0 | 2,0 | 2,0 | 2,0 | 2,0 | 2,0 |
| Distelöl | 3,0 | 3,0 | 3,0 | 3,0 | 3,0 | 3,0 |
| Controx^{®} KS | 0,05 | 0,05 | 0,05 | 0,05 | 0,05 | 0,05 |
| Baysilon^{®} M350 | 1,0 | 1,0 | 1,0 | 1,0 | 1,0 | 1,0 |
| Isopropylstearat | 6,0 | 6,0 | 6,0 | 6,0 | 6,0 | 6,0 |
| Propylparaben | 0,2 | 0,2 | 0,2 | 0,2 | 0,2 | 0,2 |
| 1,3-Butylenglycol | 5,0 | 5,0 | 5,0 | 5,0 | 5,0 | 5,0 |
| 1,6-Hexandiol | 5,0 | 5,0 | 5,0 | 5,0 | 5,0 | 5,0 |
| Methylparaben | 0,2 | 0,2 | 0,2 | 0,2 | 0,2 | 0,2 |
| Hibiscin^{®} HP LS 9198 | 3,0 | 3,0 | 3,0 | 3,0 | 3,0 | 3,0 |
| Citronensäure | 0,1 | 0,1 | 0,1 | 0,1 | 0,1 | 0,1 |
| Simulgel^{®} NS | 2,0 | 2,0 | 2,0 | 2,0 | 2,0 | 2,0 |
| Pearl Protein Extract | - | - | 2,00 | - | - | 1,00 |
| Orsirtine GL | 0,1 | 1 | 0,01 | 0,5 | 0,1 | 3 |
| Ridulisse C | 1,00 | 0,50 | 1,00 | - | - | - |
| Carnitin | 0,20 | - | - | 0,50 | 0,30 | 0,30 |
| Matrixyl 3000 | 3,00 | - | - | - | 2,00 | - |
| Aqua | ad 100 | ad 100 | ad 100 | ad 100 | ad 100 | ad 100 |

Die Zusammensetzungen 20.1 - 3 sind bevorzugte Ausführungsformen von Reinigungsgelen mit einem schützenden Effekt zur Komplettierung der Hautpflege.

| 19. Reinigungszubereitungen: | **1** | **2** | **3** | 20. Reinigungsgele | 1 | 2 | 3 |
|---|---|---|---|---|---|---|---|
| Dipropylenglycol | 10,00 | 10,00 | 10,00 | Carbomer | 1,40 | 1,40 | 1,40 |
| Chlorhexidindigluconat | 1,00 | 1,00 | 1,00 | 1,3-Butylenglycol | 4,00 | 4,00 | 4,00 |
| Synperonic PE7L 64 | 3,00 | 3,00 | 3,00 | Sodium Benzoate | 0,40 | 0,40 | 0,40 |
| D-Panthenol | 0,50 | 0,50 | 0,50 | Plantaren^{®} 1200 | 7,50 | 7,50 | 7,50 |
| Hydagen CMF | 3,00 | 3,00 | 3,00 | Dehyton^{®} K | 3,40 | 3,40 | 3,40 |
| PEG-40 Hydrogenated Castor Oil / Trideceth 9 / Propylen Glycol | 0,50 | 0,50 | 0,50 | Texapon^{®} SB 3 | 5,00 | 5,00 | 5,00 |
| Orsirtine GL | 1 | 2 | 0,1 | Cetiol^{®} HE | 0,50 | 0,50 | 0,50 |
| Parfume | 0,20 | 0,20 | 0,20 | Lamesoft^{®} PO 65 | 5,00 | 5,00 | 5,00 |
| Aqua | ad. 100 | ad. 100 | ad. 100 | Controx KS | 0,05 | 0,05 | 0,05 |
| | | | | Sodium pyrrolidone carboxylic acid | 1,60 | 1,60 | - |
| | | | | Pantolactone | 1,00 | 1,00 | - |
| | | | | Tetrasodium EDTA | 0,25 | 0,25 | 0,25 |
| | | | | Sodium | 1,80 | | |
| | | | | Lactate | | | |
| | | | | Panthenol | 0,50 | 0,50 | 0,50 |
| | | | | Orsirtine GL | 0,1 | 1 | 2 |
| | | | | Carnitin | 0,20 | - | 0,20 |
| | | | | Perfume | 0,40 | 0,40 | 0,40 |
| | | | | Aqua | ad 100 | ad 100 | ad 100 |

### 21. Beispiele für Zusammensetzungen zum Tränken von Tüchern

| | Bestandteile | 1 | 2 | 3 | 4 |
|---|---|---|---|---|---|
| Phase 1 | PEG-40 Hydrogenated Castor Oil | 0,1 | 0,1 | 0,1 | - |
| | Trideceth-9 | 0,1 | 0,1 | 0,1 | - |
| | Parfum | 0,3 | 0,1 | 0,1 | 0,1 |
| Phase 2 | Hexyllaurat | 2,0 | - | - | - |
| | Dicaprylylcarbonat | - | - | 2,0 | - |
| | Dipropylenglycol | - | 2,0 | - | - |
| Phase 3 | Allantoin | 0,5 | - | - | - |
| | Citronensäure (Monohydrat) | 1,5 | 1,5 | 1,5 | 1,5 |
| | Trinatriumcitrat (Dihydrat) | 2,0 | 2,0 | 2,0 | 2,0 |
| | Decylglucosid | - | - | - | 2,0 |
| | Pemulen TR 1 | - | - | - | 0,2 |
| | Orisitine | 1,0 | 2,0 | 2,0 | 0,1 |
| | Ridulisse C | 1,00 | 0,50 | 1,00 | - |
| | Carnitin | 0,20 | - | - | 0,50 |
| | Wasser | ad 100 | ad 100 | ad 100 | ad 100 |

Mit diesen Tränkzusammensetzungen wurden verschiedene Viskose-Vliesstoffe ausgerüstet. Es wurden gelochte, ungelochte, genoppte, einlagige, zweilagige und dreilagige Vliese ausgerüstet. Die Tücher wurden sowohl als feuchte Tücher als auch als trockene Tücher (das heißt, nach dem Ausrüsten wurden die Tücher bis auf einen Restwassergehalt kleiner 10 Gew.-%, bevorzugt kleiner 5 Gew.-%, getrocknet, um direkt vor Gebrauch mit einer kleinen Menge an Wasser wieder befeuchtet zu werden oder auf feuchter Haut angewendet zu werden) verwendet.

### 22. Matrixpflaster mit schützendem Effekt

| | 1 | 2 | 3 | 4 | 5 |
|---|---|---|---|---|---|
| DURO-TAK^{®} 387-2516 | 76 | 76 | 76 | 76 | 76 |
| Aloe Vera Gel | 1 | - | 1 | - | - |
| Propylenglycolmonooleat | 5 | 5 | - | - | - |
| Tween^{®}-80 | - | - | 5 | 3 | 5 |
| Natriumcitrat | 0,1 | 0,1 | 0,1 | 0,1 | 0,1 |
| Controx^{®} KS | 0,05 | 0,05 | 0,05 | 0,05 | 0,05 |
| Orsirtine GL | 1 | 0,1 | 0,2 | 0,5 | 2 |
| Ridulisse C | 1,00 | 0,50 | 1,00 | - | - |
| Carnitin | 0,20 | - | - | 0,50 | 0,30 |
| Matrixyl 3000 | 3,00 | - | - | - | 2,00 |
| Wasser | ad 100 | ad 100 | ad 100 | ad 100 | ad 100 |

### 23. Reservoirpflaster mit schützendem Effekt

| Bestandteile des Wirkstoffreservoirs | 1 | 2 |
|---|---|---|
| Orsirtine GL | 0,1 | 0,5 |
| Ultrasomes | 1,0 | 1,0 |
| Ridulisse C | - | 0,50 |
| Carnitin | 0,50 | - |
| Ederline H | 1,0 | 1,0 |
| Carbopol^{®} 980 | 0,5 | - |
| Pemulen^{®}TR 1 | - | 0,5 |
| NaOH | 0,1 | 0,1 |
| Titandioxid | 0,2 | 0,2 |
| Ethanol | 1,0 | 1,0 |
| Polyvinylalkohol | 2,0 | 1,0 |
| Carboxymethylcellu lose | 1,0 | 0,5 |
| Glycerin | 10 | 20 |
| Sorbitol | 7,0 | 5,0 |
| 1,3-Butandiol | 3,0 | 3,0 |
| Tween^{®}-80 | 0,05 | 0,05 |
| Paraffinöl | 5,0 | 2,0 |
| Natriumcitrat | 0,1 | 0,1 |
| Controx KS | 0,05 | 0,05 |
| Wasser | ad 100 | ad 100 |

Die Bestandteile der Wirkstoffreservoir-Rezepturen Nr. 1 bzw. 2 wurden miteinander vermischt, so dass eine gelartige Masse entstand. Mit dieser Masse wurden Pflasterträger beschichtet, so dass sogenannte Reservoirpflaster erhalten wurden. Diese Pflaster werden sowohl auf die trockene Haut als auch auf die angefeuchtete Haut gelegt und verbleiben dort für eine Zeit von wenigen Sekunden bis einigen Stunden.

### 24. Die nachfolgenden Rezepturen sind bevorzugte Beispiele für erfindungsgemäße schützende Körpercremes:

| | | |
|---|---|---|
| Paraffinum Liquidum | 20,00 | 20,00 |
| Arlacel 165 | 4,00 | 4,00 |
| Eutanol G | 1,00 | 1,00 |
| Cetiol SN | 1,50 | 1,50 |
| Cremophor A 6 | 2,00 | 2,00 |
| Behenylalkohol | 1,60 | 1,60 |
| Silikonöl 350 cs | 3,00 | 3,00 |
| Brij 721 VP | 0,40 | 0,40 |
| Propylparaben | 0,20 | 0,20 |
| Controx KS | 0,25 | 0,25 |
| Propandiol-1,2 | 3,00 | 3,00 |
| Glycerin 86% pflanzlich | 5,00 | 5,00 |
| Polyethylenglykol MG 400 | 1,70 | 1,70 |
| Methylparaben | 0,20 | 0,20 |
| Phenoxyethanol, rein | 0,50 | 0,50 |
| Carbopol ETD 2020 | 0,50 | 0,50 |
| Natriumhydroxid | 0,03 | 0,03 |
| Citronensäure Monohydrat | 0,10 | 0,10 |
| Trinatriumcitrat Dihydrat | 0,10 | 0,10 |
| Orsirtine GL | 1 | 0,1 |
| Sensiva SC 50 (Octoxyglycerin) | 0,50 | 0,50 |
| Carnitin | 0,20 | 0,20 |
| Ergothionein | - | 0,10 |
| Parfum | 0,30 | 0,30 |
| Simulgel NS | 1,00 | 1,00 |
| Wasser | ad 100,00 | ad 100,00 |

### 25. Sonnenschutzrezepturen (Öl-in-Wasser-Emulsionen) [alle Mengenangaben in Gew.-%]

| | Nr. 1 | Nr. 2 | Nr. 3 | Nr. 4 |
|---|---|---|---|---|
| Caprylic/ Capric Triglycerid | 3,5 | 3,5 | 3,5 | 3,5 |
| Distelöl | 2 | 2 | 2 | 2 |
| Cetiol B | 5 | 5 | 5 | 5 |
| Cetiol Sensoft | 1 | 1 | 1 | 1 |
| Behenylalkohol | 3 | 3 | 3 | 3 |
| Tocopherylacetat | 0,5 | 0,5 | 0,5 | 0,5 |
| Controx KS | 0,05 | 0,05 | 0,05 | 0,05 |
| Parsol 1789 | 3 | 3 | 3 | 3 |
| Parsol SLX | 3 | 3 | 3 | 3 |
| Propylparaben | 0,2 | 0,2 | 0,2 | 0,2 |
| Hexandiol-1,6 | 5 | 5 | 5 | 5 |
| Glycerin, 86% | 4,5 | 4,5 | 4,5 | 4,5 |
| Sorbit, 70% | 2 | 2 | 2 | 2 |
| Neo Helio-pan Hydro | 3 | 3 | 3 | 3 |
| AMP | 0,99 | 0,99 | 0,99 | 0,99 |
| NaOH (fest) | 0,365 | 0,365 | 0,365 | 0,365 |
| Emulsiphos 677660 | 3 | 3 | 3 | 3 |
| Methylparaben | 0,2 | 0,2 | 0,2 | 0,2 |
| Bienen-wachs | 0,2 | 0,2 | 0,2 | 0,2 |
| Cosmedia SP | 0,5 | 0,5 | 0,5 | 0,5 |
| Tego Carbomer 140 | 0,5 | 0,5 | 0,5 | 0,5 |
| NTA-Na₃, 40% | 0,1 | 0,1 | 0,1 | 0,1 |
| Silica | 2 | 2 | 2 | 2 |
| Orsirtine GL | 0,1 | 0,2 | 1 | 2 |
| Wasser | 51,16 | 51,188 | 56,217 | 56,217 |

### Liste der verwendeten Rohstoffe

| Handelsname | INCl-Bezeichnung | Lieferant/Hersteller |
|---|---|---|
| Abil EM 90 | CETYL PEG/PPG-1 0/1 DIMETHICONE | Degussa |
| Argireline | Acetyl-Hexapeptide-3 | Lipotec |
| Arlacel 165 | Glyceryl Stearate, PEG-100 Stearate | Uniqema |
| AMP | Aminomethyl propanol (2-Amino-2-methylpropanol) | Dow |
| Aristoflex AVC | Ammonium Acryloly-Dimethyltaurat/VP Copolymer, t-Butyl-Alcohol | Clariant |
| Baysilone-Öl M 350 | Dimethicone | GE Bayer Silicones |
| Belsil DM 100 | Dimethicone | Wacker |
| Brij 721 | STEARETH-21 | Uniqema |
| Calmosensine | Butylene glycol, Water, LAURETH-3, HYDROXY- ETHYLCELLULOSE, Acetyl Dipeptide-1 Cetylester | Sederma |
| Caomint | Propylene glycol, Water, Menta piperita (PEPPERMINT) LEAF EXTRACT, THEOBROMA CACAO (COCOA) EXTRACT | Solatia |
| Carbopol 980 | Carbomer | Noveon |
| Carbopol ETD 2020 | Acrylates/C10-30 Alkyl Acrylate Crosspolymer | Noveon |
| Cetiol^{®} 868 | Ethylhexyl stearate (vegetable base) | Cognis |
| Cetiol^{®} B | Dibutyl Adipate | Cognis |
| Cetiol^{®} CC | Dioctyl carbonate | Cognis |
| Cetiol^{®} HE | PEG-7 Glyceryl Cocoate | Cognis |
| Cetiol^{®} SB 45 | Butyrospermum parkii (Shea butter) | Cognis |
| Cetiol^{®} Sensoft | Propylheptyl Caprylate (2-Propylheptyl octanoate) | Cognis |
| Cetiol^{®} SN | Cetearyl Isononanoate | Cognis |
| Controx KS | Tocopherol, Hydrogenated Palm Glycerides Citrate | Cognis |
| Cosmedia SP | Sodium polyacrylate | Cognis |
| Cremophor A 6 | CETEARETH-6, STEARYL ALCOHOL | BASF |
| Culminal MHPC 100 | Hydroxypropylmethylcellulose | Hercules |
| Cutina CBS | Glyceryl Stearate / Cetearyl Alcohol / Cetyl alpitate / Cocoglycerides im Gewichtsverhältnis 7:1:1:1 | Cognis |
| Cutina GMS-V | Glyceryl Stearate (Mischung aus Glycerylmono- und distearat) | Cognis |
| Cutina MD | Glyceryl Stearate (Mischung aus Glycerylmono- und distearat) | Cognis |
| Dehyton K | Water, Cocamidopropyl betaine (29 - 32 Gew.-%) | Cognis |
| Deliner | Zea Mays (Corn) Kernel Extract, Butylene Glycol, Xanthan Gum | Coletica |
| Dow Corning 1403 Fluid | Cyclomethicone (86 - 88 Gew.-%), Dimethiconol (12 - 14 Gew.-%) | Dow Corning |
| Dow Corning 1501 Fluid | Cyclomethicone (85 - 86 Gew.-%), Dimethiconol (14-15 Gew.-%) | Dow Corning |
| Dow Corning 200 Fluid | Dimethicone | Dow Corning |
| Dow Corning 9040 | Cyclomethicone/ Dimethicone Crosspolymer (87- 88%/12-13 %) | Dow Corning |
| Dow Corning245 Fluid | Cyclomethicone | Dow Corning |
| Dry Flo Plus | Aluminium Starch Octenylsuccinate | National Starch |
| DSH CN | Water, Dimethylsilanol Hyaluronate | Exsymol |
| DURO-TAK^{®} 387-2516 | Polyacrylate, Ethylcellulose, Polyvinylpyrrolidon | National Starch and Chemical |
| Estasan GT98-60 3575 MCT Oil | Echium Plantagineum Seed Oil | Croda |
| Ederline H | PEG-40 Hydrogenated Castor Oil, PPG-2-Ceteareth-9, Pyrus Malus (Apple) Fruit Extract | Sporga |
| Ederline L bzw. LS | Hexyldecanol, Pyrus Malus (Apple) Fruit Extract | Seporga |
| Elfacos ST 37 | PEG-45/Dodecyl Glycol Copolymer | Akzo Nobel |
| Emulsiphos 677660 | Potassium Cetyl Phosphate, Hydrogenated Palm Glycerides | Symrise |
| Eumulgin B 3 | Ceteareth-30 | Cognis |
| Eutanol G | OCTYLDODECANOL | Cognis |
| Fucogel 1000 | Biosaccharide Gum-1 | Solatia |
| Generol R | Brassica Campestris (RAPESEED) Sterols | Cognis |
| Glistin | Water, L-Glutamylaminoethyl indole | Exsymol |
| Glucamate Sse-20 | PEG-20 methylglucose sesquistearate | Noveon |
| Hibiscin^{®} HP-LS-9198 | Water, Hibiscus esculentus Seed Extract, Phenoxyethanol | Laboratoires Sérobiologiques |
| Hydagen CMF | Chitosan Glycolate | Cognis |
| Hydrovance | Wasser, Bis-N,N'-(2-Hydroxyethyl)harnstoff, Harnstoff, Ammoniumlactat | National Starch |
| Keltrol SF | Xanthan Gum | Kelco |
| Keratec Pep | Water, Hydrolyzed Keratin | Croda |
| Kombuchka | Saccharomyces/Xylinum/Black Tea Ferment, Glycerin, Hydroxyethylcellulose | Sederma |
| Lameform TGI | Polyglyceryl-3 Diisostearate | Cognis |
| Lamesoft^{®} PO 65 | Coco-Glucoside, Glyceryl Oleate, Water | Cognis |
| LANETTE^{®} 22 | Behenyl Alcohol | Cognis |
| Liftiline | Aqua, Hydrolyzed Wheat Protein (7 - 10 Gew.-% Aktivsubstanz) | Silab |
| Lipochroman 6 | DIMETHYLMETHOXY CHROMANOL | Lipotec |
| Lipoid S 75-3 | Aqua, Lecithine | Lipoid GmbH |
| Matrixyl | Aqua, Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Montanov 68 | Cetearyl Alkohol, Cetearyl Glucoside | Seppic |
| Montanov 202 | Arachidyl Alkohol, Behenyl Alcohol, Arachidyl Glucoside | Seppic |
| Myritol 318 | Caprylic/Capric Triglyceride | Cognis |
| MYRITOL^{®} PC | Propylene Glycol, DICAPRYLATE/DICAPRATE | Cognis |
| Natipide 2 PG | Aqua, Propylene Glycol, Lecithin | Phospholipid GmbH |
| Natrulon RC 50 DG | WATER, CARNITINE, POLYGLYCERIN-10 | Lonza |
| Neo Heliopan AP | Disodium Phenyl Dibenzimidazole Tetrasulfonate | Symrise |
| Novata AB | Cocoglycerides | Cognis |
| Parsol 1789 | BUTYL METHOXYDIBENZOYLMETHANE | DSM |
| Parsol 340 | Octocrylene | DSM |
| Parsol HS | PHENYLBENZIMIDAZOLE SULFONIC ACID | DSM |
| Parsol SLX | Polysilicone-15 | DSM |
| Pearl Protein Extract | Aqua, Hydrolyzed Conchiolin Protein | Maruzen |
| Pemulen TR 1 | Acrylates/C10-30 Alkyl Acrylate Crosspolymer | Noveon |
| Photosomes | Aqua, Lecithine, Plancton Extract | AGI Dermatics |
| Phytokine | Aqua, Butylene Glycol, HYDROLYZED SOY PROTEIN, Methylparaben, Ethylparaben, Propylparaben (0,4 - 0,8 Gew.-% Aktivsubstanz) | Coletica |
| Plantaren^{®} 1200 | Lauryl Glucoside, ca. 50 % Aktivsubstanz | Cognis |
| Orsirtine^{®} GL | Aqua, Glycerol, Oryza Sativa (Rice) Extract, 0,4 Gew.-% Aktivsubstanz | Vinciences |
| Relipidium AHYA017B | Hydrolyzed Yeast Protein, Butylene Glycol, Methylparaben | Coletica |
| Rhamnosoft | Biosaccharide Gum-2 | Solabia |
| Ridulisse C | Water, HYDROLYZED SOY PROTEIN (3 - 5 Gew.-% Aktivsubstanz) | Silab |
| Ronasphere^{®} LDP | SILICA, Cl 77891 (TITANIUM DIOXIDE), Cl 77491 (IRON OXIDES) | Merck KGaA |
| Sensiva^{®} SC 50 | 2-Ethylhexylglycerinether | Schülke & Mayr |
| Sepigel^{®}305 | Aqua (Water) Polyacrylamide, C₁₃-C₁₄ Isoparaffin, Laureth-7 (Aktivsubstanz Verdickerpolymer ca. 45 - 49 Gew.-%) | SEPPIC |
| Sepilift DPHP | Dipalmitoyl Hydroxyproline | Seppic |
| Sepivinol R | Wine Extract (Polyphenolreicher Extrakt aus Rotwein) | Seppic |
| Silymarin Phytosome | Silybum Marianum Extract and Phospholipids | Indena SpA |
| Simulgel EPG | Aqua (Water)/Sodium Acrylate / Sodium Acryloyldimethyl Taurate Copolymer / Polyisobutene / Caprylyl/Capryl Glucoside | Seppic |
| Simulgel NS | Aqua (Water)/Hydroxyethyl Acrylate / Sodium Acryloyl-dimethyl Taurate Copolymer / Squalane / Polysorbate 60 | Seppic |
| Sisterna SP 30 C | Sucrosedistearate | Sisterna B.V. |
| Sisterna SP 70 C | Sucrose stearate/palmitate | Sisterna B.V. |
| Softisan 649 | Bis-Diglyceryl Polyacyladipate-2 | Sasol |
| Spirulina 3002 | Water, Algae extract (Spirulina platensis) | Interorgana |
| Stenol 16/18 | Cetearylalkohol | Cognis |
| Symdiol 68 | 1,2-Octandiol, 1,2-Hexandiol | Symrise |
| Sympatens-BS/080 | Stearic acid ethoxylated (8 EO) | Dr. W. Kolb |
| Synperonic PE L 64 | Poloxamer-184 | Uniqema |
| Tego Carbomer 140 | Carbomer | Degussa |
| Texapon^{®} SB 3 | Aqua, Disodium Laureth Sulfosuccinate, (ca. 40 % Aktivsubstanz), Citric Acid | Cognis |
| Tween^{®}-80 | Polysorbate-80 | |
| Ultrasomes | Aqua, Lecithine, Micrococcus Luteus Extract | AGI Dermatics |
| Uvinul T 150 | ETHYLHEXYL TRIAZONE | BASF |
| Uvinul MBC 95 | 4-METHYLBENZYLIDENE CAMPHOR | BASF |

## Patentansprüche

1. Kosmetische, nicht-therapeutische Verwendung eines Reisextrakts zur Verringerung der Porengröße der Haut, insbesondere der Gesichtshaut.

2. Verwendung nach Anspruch 1, **dadurch gekennzeichnet, dass** die Porengröße um mindestens 3 %, bevorzugt mindestens 5 %, insbesondere um mindestens 7 % und ganz besonders bevorzugt um mindestens 10 % der Ausgangsporengröße verringert wird.

3. Verwendung nach einem der Ansprüche 1 bis 2, **dadurch gekennzeichnet, dass** der Extrakt durch Extraktion von Reiskörnern, insbesondere ausgewählt aus Körner von Oryza sativa L. und Oryza glaberrima Steud., bevorzugt Oryza sativa L., mit Extraktionsmitteln ausgewählt aus Wasser, wässrige Lösungen, ein- oder mehrfachen Alkoholen mit einer Kettenlänge von 1 bis 6 Kohlenstoffatomen sowie Gemischen aus zwei oder mehreren der genannten Extraktionsmitteln, hergestellt wird.

4. Verwendung nach einem der Ansprüche 1 bis 3, **dadurch gekennzeichnet**, der Extrakt Reisproteine und/oder Proteinhydrolysate enthält.

5. Verwendung nach einem der Ansprüche 1 bis 4, **dadurch gekennzeichnet, dass** der Reisextrakt mindestens 20 Gew.-%, bevorzugt mindestens 25 Gew.-%, insbesondere mindestens 35 Gew.-% und ganz besonders bevorzugt 41 Gew.-% Peptide (bezogen auf die Trockenmasse des Extrakts) enthält.

6. Verwendung nach einem der Ansprüche 1 bis 5, **dadurch gekennzeichnet, dass** der Reisextrakt in einer Zusammensetzung verwendet wird, die den Reisextrakt in einer Gesamtmenge von 0,00001 - 10 Gew.-%, bevorzugt 0,0001 - 1 Gew.-%, besonders bevorzugt 0,002 - 0,1 Gew.-%, außerordentlich bevorzugt 0,003 - 0,05 Gew.-%, jeweils bezogen auf den Gehalt an Aktivsubstanz des Extrakts in der gesamten Zusammensetzung enthält.

7. Verwendung nach einem der Ansprüche 1 bis 6, **dadurch gekennzeichnet, dass** der Reisextrakt in Kombination mit mindestens einen Anti-Aging Wirkstoff verwendet wird.

8. Verwendung nach Anspruch 7, **dadurch gekennzeichnet, dass** der mindestens eine Anti-Aging Wirkstoff ausgewählt ist aus Apfelkernextrakten (Pyrus Malus (Apple) Fruit Extract), Hyaluronsäure und/oder ihren Salzen, Lotuskeim-Extrakten (Nelumbo Nucifera Germ Extract), Extrakten aus Maiskörnern (Zea Mays (Corn) Kernel Extract), Extrakten aus Schwarzen Holunderblüten (Sambucus Nigra Flower Extract), Mischungen aus mindestens einem Extrakt aus Kakaobohnen (Theobroma cacao) und mindestens einem Extrakt aus den Blättern der Pfefferminze (Mentha piperita), Resveratrol und/oder Resveratrolmono-, - di- und -triphosphorsäureestern und deren Salzen und/oder Resveratrolmono-, -di- und - trimethylether, Isoflavonoiden und Isoflavonoid-reichen Pflanzenextrakten, Dihydroquercetin (= Taxifolin) sowie Mischungen hiervon, insbesondere aus Hyaluronsäure, einem oder mehreren Salzen der Hyaluronsäure und/oder Apfelkernextrakten.

9. Verwendung nach einem der Ansprüche 1 bis 8, **dadurch gekennzeichnet, dass** der Reisextrakt in Kombination mit einem Antioxidans ausgewählt aus substituierten oder nicht substituierten 2,2-Dialkylchroman-Derivaten, bevorzugt ausgewählt aus Tocopherolen, Tocopherylacetat oder 6,7-disubstituiertes 2,2-Dialkylchroman oder -chromen, besonders bevorzugt Tocopherylacetat und Dimethylmethoxy Chromanol eingesetzt wird.

10. Verwendung nach einem der Ansprüche 1 bis 9, **dadurch gekennzeichnet, dass** der Reisextrakt in Kombination mit wenigstens einem weiteren, vorzugsweise mehreren, Inhaltsstoffe eingesetzt wird, ausgewählt ausgewählt aus mindestens einem sebumregulierenden Wirkstoff, mindestens einem partikelförmigen, sebumsorbierenden Wirkstoff und mindestens einem adstringierenden Wirkstoff, Absorptionsmitteln, antimikrobiellen Stoffen, Stoffen biologischen Ursprungs, Chelatbildnern, Emollientien, Emulgatoren/Dispergatoren, Emulsionsstabilisatoren, Feuchtigkeitsspendern, Filmbildnern, Konservierungsstoffen, hautkühlenden Wirkstoffen, Tensiden/waschaktiven Substanzen, Treibgasen, Trübungsmitteln, mindestens ein Vitamin, Provitamin oder eine als Vitaminvorstufe bezeichnete Verbindung aus den Vitamingruppen A, B, H und K und den Estern der vorgenannten Substanzen Hydrogelbildnern, Lösungsmitteln, mindestens einem alkyl-oder hydroxyalkylsubstituierten Harnstoff der allgemeinen Formel (A), 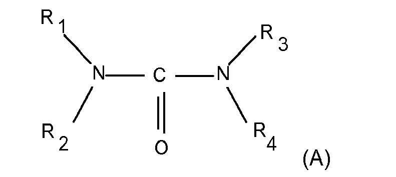 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-HydroxyalkylGruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt, weiterhin ausgewählt aus mindestens einem weiteren kosmetischen Wirkstoff, der ausgewählt ist aus Monomeren, Oligomeren und Polymeren von Aminosäuren, N-C₂-C₂₄-Acylaminosäuren und/oder den Estern und/oder den physiologisch verträglichen Metallsalzen dieser Substanzen, sowie Mischungen dieser Wirkstoffe, mindestens einem DNA-Oligonucleotid oder mindestens einem RNA-Oligonucleotid und mindestens einer natürlichen Betainverbindung.

11. Kosmetische Zusammensetzung, enthaltend
a) mindestens einen Reisextrakt und
b) mindestens ein Salz von C₁₂₋₂₀-Alkylphosphat, insbesondere ein Salz von Cetylphosphat.

12. Kosmetische Zusammensetzung nach Anspruch 11, **dadurch gekennzeichnet, dass** weiterhin mindestens ein UV-Filter, ausgewählt aus öllöslichen und wasserlöslichen organischen UV-Filtern, enthalten ist.

13. Kosmetische Zusammensetzung nach Anspruch 12, **dadurch gekennzeichnet, dass** der mindestens eine wasserlösliche organische UV-Filter mindestens ein Derivat der Benzimidazolsulfonsäure umfasst.

14. Kosmetische Zusammensetzung nach Anspruch 12, **dadurch gekennzeichnet, dass** der mindestens eine öllösliche organische UV-Filter Polysilicone-15 umfasst.

15. Verfahren zur Verringerung der Porengröße der Haut, **dadurch gekennzeichnet, dass** ein Reisextrakt oder eine Zusammensetzung, enthaltend einen Reisextrakt, auf die Haut aufgetragen wird.
